# EUROPEAN PATENT APPLICATION

(11) **EP 4 245 342 A1**
(43) Date of publication of application: **20.09.2023**
(21) Application number: 20967892.9
(22) Date of filing: 31.12.2020
(51) Int. Cl.: A61M 16/01

(54) **MEDICAL ALARM SYSTEM, METHOD AND DEVICE, ANESTHESIA DEVICE AND WEIGHING DEVICE**

(71) Applicant: Shenzhen Mindray Animal Medical Technology Co., Ltd., Shenzhen, Guangdong 518110 (CN)
(72) Inventor: CHEN, Peitao, Shenzhen, Guangdong 518057 (CN); CHEN, Yiwei, Shenzhen, Guangdong 518057 (CN)
(74) Representative: KIPA AB
(86) International application number: PCT/CN2020/142476
(87) International publication number: WO 2022/141554

(57) **Abstract**

Provided in embodiments of the disclosure is a medical alarm system, including an anesthesia device, a weigher and a waste gas adsorption canister, wherein the waste gas adsorption canister is connected to the anesthesia device; the anesthesia device is in mutual communication with the weigher; the anesthesia device is configured with a first alarm apparatus thereon, wherein the anesthesia device is configured to release an anesthetic gas and discharge a waste anesthetic gas; the waste gas adsorption canister is configured to adsorb the waste anesthetic gas; the weigher is configured to acquire weight data of the waste gas adsorption canister by weighing in real time and transmit the weight data to the anesthesia device; the anesthesia device is configured to determine whether to trigger an alarm based on the weight data and at least one preset alarm threshold, and output at least one alarm prompt of sound, flashing light and an interface prompt by the first alarm apparatus upon determining to trigger an alarm, wherein the at least one preset alarm threshold is a data criterion for an adsorption capacity abnormality of the waste gas adsorption canister or a data abnormality of the weigher. The intelligence and safety of the medical alarm system can be improved by the embodiments of the disclosure.

## Description

### TECHNICAL FIELD

Embodiments of the disclosure relate to the technical field of medical instruments, and more particularly, to a medical alarm system, method and device, an anesthesia device, and a weigher.

### BACKGROUND

Low-flow inhalation anesthesia is a commonly used anesthesia method at present. When anesthesia is applied, except anesthetic inhaled by an anesthesia subject, an excess gas is discharged through a waste gas outlet of an anesthesia machine. In order to ensure ambient safety and physical health of medical care personnel, an activated carbon adsorption canister is used in most animal hospitals, which is connected to a waste gas discharge outlet of the anesthesia machine and configured to adsorb anesthetic in the waste gas and to discharge a filtered gas. The activated carbon adsorption canister has a limited adsorption capacity and needs to be replaced in time. At present, a low-cost and easy-to-implement weighing method is widely used to give an alarm: providing a weigher to weigh the activated carbon adsorption canister, and transmitting by the weigher, alarm information such as light or sound to remind of replacement.

However, when the weighing method is adopted, an external weigher is generally placed on a back side of the anesthesia machine, which results in poor effectiveness of alarming by the weigher. Also, with limitations of its hardware conditions, the weigher cannot meet standard specifications of acoustic and optical alarms, and thus the safety of alarm prompts is reduced. In addition, the weigher can only detect a single abnormal scenario and has limited functions, and has difficulties in detecting and alerting a variety of abnormal situations which may occur in the activated carbon adsorption canister and in further eliminating the abnormal situations, and the degree of intelligence is low.

### SUMMARY

Embodiments of the disclosure are intended to provide a medical alarm system, method and device, an anesthesia device, and a weigher, which are capable of improving the safety and intelligence of the medical alarm system.

The technical solutions of the embodiments of the disclosure may be implemented as follows:
An embodiment of the disclosure provides a medical alarm system, including an anesthesia device, a weigher and a waste gas adsorption canister; wherein the waste gas adsorption canister is connected to the anesthesia device, and the waste gas adsorption canister is placed in a preset weight measurement region of the weigher; the anesthesia device is in mutual communication with the weigher; the anesthesia device is configured with a first alarm apparatus thereon, wherein the anesthesia device is configured to release an anesthetic gas and to discharge a waste anesthetic gas; the waste gas adsorption canister is configured to adsorb the waste anesthetic gas; the weigher is configured to acquire weight data of the waste gas adsorption canister by weighing in real time and to transmit the weight data to the anesthesia device; and the anesthesia device is configured to determine whether to trigger an alarm based on the weight data and at least one preset alarm threshold, and output an alarm prompt by means of the first alarm apparatus when it is determined to trigger an alarm; wherein the at least one preset alarm threshold is a data criterion for an adsorption capacity abnormality of the waste gas adsorption canister or a data abnormality of the weigher; and the alarm prompt includes at least one of sound, flashing light and an interface prompt.

In the above-mentioned system, the at least one preset alarm threshold includes: a first preset weight threshold; and
the anesthesia device is further configured to determine to trigger an alarm and output a first alarm prompt if the weight data is greater than the first preset weight threshold.

In the above-mentioned system, the anesthesia device is configured with a human-machine interaction device thereon which includes a display, wherein the human-machine interaction device is configured to display a preset weight setting control on a preset anesthesia interface by means of the display, and to receive input data by means of the preset weight setting control; and the anesthesia device is further configured to determine the first preset weight threshold based on the input data.

In the above-mentioned system, the at least one preset alarm threshold includes a second preset weight threshold; wherein the anesthesia device is further configured to: take the currently received weight data as startup weight data for each time ventilation is initiated, to determine whether the startup weight data exceeds the second preset weight threshold, wherein the second preset weight threshold is smaller than the first preset weight threshold, and to determine to trigger an alarm and output a second alarm prompt if the startup weight data is greater than the second preset weight threshold.

In the above-mentioned system, the at least one preset alarm threshold includes a preset slope threshold; wherein the anesthesia device is further configured to: record corresponding time information of acquiring the weight data; generate a slope of weight increase of the waste gas adsorption canister based on the weight data and the time information, wherein the slope of weight increase represents the trend of weight increase of the waste gas adsorption canister; acquire a current ventilation parameter in real time; and determine to trigger an alarm and output the first alarm prompt if the current ventilation parameter is not changed and the slope of weight increase is smaller than the preset slope threshold within a preset time period.

In the above-mentioned system, the anesthesia device is further configured to obtain a residual usage amount of the waste gas adsorption canister based on the first preset weight threshold and the current ventilation parameter, and to display the residual usage amount by means of the display in real time.

In the above-mentioned system, the at least one preset alarm threshold includes a preset weight range; wherein the anesthesia device is further configured to determine to trigger an alarm and output a third alarm prompt if the weight data transmitted by the weigher is not received within a first preset time interval or the received weight data exceeds the preset weight range, wherein the third alarm prompt is configured to prompt that the weigher is faulty.

In the above-mentioned system, the anesthesia device is further configured to, upon triggering an alarm, save a current alarm event in a preset alarm record list; and the display is further configured to, upon receiving a preset view instruction, display the preset alarm record list in response to the preset view instruction.

In the above-mentioned system, the preset weight setting control is a selection input control, wherein the human-machine interaction device is further configured to receive target specification information by means of the selection input control; and the anesthesia device is further configured to determine a preset weight threshold corresponding to the target specification information as the first preset weight threshold according to the target specification information and based on a preset correspondence between at least one term of preset specification information and at least one preset weight threshold.

In the above-mentioned system, the display is further configured to display a preset initialization control on a preset anesthesia interface; the human-machine interaction device is further configured to, upon receiving an initialization trigger operation for the preset initialization control, send a weighing initialization instruction to the weigher in response to the initialization trigger operation; and the weigher is further configured to, upon receiving the weighing initialization instruction, take currently acquired weight data as a reference weighing value, so as to take a weight of the waste gas adsorption canister increased based on the reference weighing value as the weight data.

In the above-mentioned system, the weigher is configured with a second alarm apparatus; the anesthesia device is further configured to send an alarm instruction to the weigher when it is determined to trigger an alarm; and the weigher is configured to give an alarm by means of the second alarm apparatus upon receiving the alarm instruction.

An embodiment of the disclosure provides a medical alarm system, including an anesthesia device, a weigher and a waste gas adsorption canister; wherein the waste gas adsorption canister is connected to the anesthesia device, and the waste gas adsorption canister is placed in a preset weight measurement region of the weigher; the anesthesia device is in mutual communication with the weigher; the anesthesia device is configured with a first alarm apparatus thereon, wherein the anesthesia device is configured to release an anesthetic gas and to discharge a waste anesthetic gas; the waste gas adsorption canister is configured to adsorb the waste anesthetic gas; the weigher is configured to acquire weight data of the waste gas adsorption canister by weighing in real time, and to perform processing and analysis based on the weight data and at least one preset alarm threshold to obtain an analysis result, and to send the analysis result to the anesthesia device, wherein the analysis result indicates whether an adsorption capacity of the waste gas adsorption canister is abnormal or whether the data of the weigher is abnormal; and the anesthesia device is configured to determine whether to trigger an alarm based on the analysis result, and to output an alarm prompt by means of the first alarm apparatus when it is determined to trigger an alarm, wherein the alarm prompt includes at least one of sound, flashing light and an interface prompt.

In the above-mentioned system, the at least one preset alarm threshold includes a first preset weight threshold; wherein the weigher is further configured to send the analysis result indicating that the adsorption canister is overweight, to the anesthesia device, if the weight data is greater than the first preset weight threshold; and the anesthesia device is further configured to determine to trigger an alarm and output a first alarm prompt if the analysis result is that the adsorption canister is overweight.

In the above-mentioned system, the weigher is configured with a weight setting apparatus thereon, and the weight setting apparatus is configured to receive the first preset weight threshold.

In the above-mentioned system, the at least one preset alarm threshold includes a second preset weight threshold; wherein the anesthesia device is further configured to send a ventilation initiation instruction to the weigher each time ventilation is initiated; the weigher is further configured to, upon receiving the ventilation initiation instruction, take the currently acquired weight data as startup weight data to determine whether the startup weight data exceeds the second preset weight threshold; and send the analysis result indicating that an available usage amount of the adsorption canister is insufficient, to the anesthesia device, if the startup weight data is greater than the second preset weight threshold, wherein the second preset weight threshold is smaller than the first preset weight threshold; and the anesthesia device is further configured to determine to trigger an alarm and output a second alarm prompt if the analysis result is that the available usage amount of the adsorption canister is insufficient.

In the above-mentioned system, the at least one preset alarm threshold includes a preset slope threshold; wherein the weigher is further configured to: acquire a current ventilation parameter from the anesthesia device in real time; record corresponding time information of acquiring the weight data; generate a slope of weight increase of the waste gas adsorption canister based on the weight data and the time information, wherein the slope of weight increase represents a historical trend of weight increase of the waste gas adsorption canister; and send the analysis result indicating that the adsorption canister is overweight to the anesthesia device, if the current ventilation parameter is not changed and the slope of weight increase is smaller than the preset slope threshold within a preset time period; and the anesthesia device is further configured to determine to trigger an alarm and output the first alarm prompt if the analysis result is that the adsorption canister is overweight.

In the above-mentioned system, the anesthesia device is configured with a display; the weigher is further configured to obtain a residual usage amount of the waste gas adsorption canister based on the first preset weight threshold and the current ventilation parameter, and to send the residual usage amount to the anesthesia device; and the anesthesia device is further configured to display the residual usage amount by means of the display in real time.

In the above-mentioned system, the at least one preset alarm threshold includes a preset weight range; the weigher is further configured to determine that the analysis result is that the weigher is faulty, if the acquired weight data exceeds the preset weight range; and the anesthesia device is further configured to trigger an alarm and output a third alarm prompt, if the analysis result is not received within a second preset time interval or the analysis result is that the weigher is faulty.

In the above-mentioned system, the anesthesia device is further configured to, upon triggering an alarm, save a current alarm event in a preset alarm record list; and the display is further configured to, upon receiving a preset view instruction, display the preset alarm record list in response to the preset view instruction.

In the above-mentioned system, an information input control is preset on the weight setting apparatus; the information input control is configured to receive target specification information; and the weigher is further configured to determine a preset weight threshold corresponding to the target specification information as the first preset weight threshold according to the target specification information and based on a preset correspondence between at least one term of preset specification information and at least one preset weight threshold.

In the above-mentioned system, the weigher is further configured with a preset initialization button, wherein the weigher is further configured to, upon receiving a trigger operation for the preset initialization button, take currently acquired weight data as a reference weighing value in response to the trigger operation, so as to take a weight of the waste gas adsorption canister increased based on the reference weighing value as the weight data.

In the above-mentioned system, the weigher is configured with a second alarm apparatus; and the second alarm apparatus is configured to output an alarm prompt if the analysis result is any one of the adsorption canister being overweight, the available usage amount of the adsorption canister being insufficient and the weigher being faulty.

An embodiment of the disclosure provides a medical alarm method, including:
receiving weight data acquired and sent by a weigher in real time; and determining whether to trigger an alarm based on the weight data and at least one preset alarm threshold, and outputting an alarm prompt when it is determined to trigger an alarm, wherein the alarm prompt includes at least one of sound, flashing light and an interface prompt, and the at least one preset alarm threshold is a data criterion for an adsorption capacity abnormality of a waste gas adsorption canister or a data abnormality of the weigher.

In the above-mentioned method, the at least one preset alarm threshold includes a first preset weight threshold, and determining whether to trigger an alarm based on the weight data and at least one preset alarm threshold and outputting an alarm prompt when it is determined to trigger an alarm include:
determining to trigger an alarm and outputting a first alarm prompt if the weight data is greater than the first preset weight threshold.

In the above-mentioned method, the anesthesia device is configured with a human-machine interaction device thereon which includes a display; and before determining whether to trigger an alarm based on the weight data and at least one preset alarm threshold, the method further includes:
displaying a preset input control on a preset anesthesia interface by means of the display; receiving input data by means of the preset input control; and determining the first preset weight threshold based on the input data.

In the above-mentioned method, the at least one preset alarm threshold includes a second preset weight threshold; and determining whether to trigger an alarm based on the weight data and at least one preset alarm threshold and outputting an alarm prompt when it is determined to trigger an alarm include:
taking currently received weight data as startup weight data for each time ventilation is initiated, to determine whether the startup weight data exceeds the second preset weight threshold, wherein the second preset weight threshold is smaller than the first preset weight threshold; and
determining to trigger an alarm and output a second alarm prompt if the startup weight data is greater than the second preset weight threshold.

In the above-mentioned method, the at least one preset alarm threshold includes a preset slope threshold, and determining whether to trigger an alarm based on the weight data and at least one preset alarm threshold and outputting an alarm prompt when it is determined to trigger an alarm include:
recording time information corresponding to the weight data;
generating a slope of weight increase of the waste gas adsorption canister based on the weight data and the time information, wherein the slope of weight increase represents a historical trend of weight increase of the waste gas adsorption canister;
acquiring a current ventilation parameter in real time; and
determining to trigger an alarm and output a first alarm prompt if the current ventilation parameter is not changed and the slope of weight increase is smaller than the preset slope threshold within a preset time period.

In the above-mentioned method, after acquiring the current ventilation parameter in real time, the method further includes: obtaining a residual usage amount of the waste gas adsorption canister based on the first preset weight threshold and the current ventilation parameter; and displaying the residual usage amount by means of the display in real time.

In the above-mentioned method, the at least one preset alarm threshold includes a preset weight range; and before obtaining the weight data acquired and transmitted by the weigher in real time, the method further includes:
determining to trigger an alarm and outputting a third alarm prompt if the weight data is not received within a preset time interval, wherein the third alarm prompt represents that the weigher is faulty;
alternatively, after obtaining the weight data acquired and transmitted by the weigher in real time, the method further includes:
   determining to trigger an alarm and outputting a third alarm prompt if the weight data exceeds a preset numerical value range.

In the above-mentioned method, after determining whether to trigger an alarm based on the weight data and at least one preset alarm threshold and outputting an alarm prompt when it is determined to trigger an alarm, the method further includes: saving a current alarm event in a preset alarm record list upon triggering an alarm; and upon receiving a preset view instruction, displaying the preset alarm record list in response to the preset view instruction.

In the above-mentioned method, the preset weight setting control is a selection input control; receiving input data by means of the preset input control includes:
receiving target specification information as the input data by means of the selection input control; and
determining the first preset weight threshold based on the input data includes:
   if the input data is the target specification information, determining a preset weight threshold corresponding to the target specification information as the first preset weight threshold based on a preset correspondence between at least one term of preset specification information and at least one preset weight threshold.

The above-mentioned method further includes:
displaying a preset initialization control on a preset anesthesia interface; and
upon receiving an initialization trigger operation for the preset initialization control, sending a weighing initialization instruction to the weigher in response to the initialization trigger operation, such that the weigher, upon receiving the weighing initialization instruction, takes currently acquired weight data as a reference weighing value, so as to take a weight of the waste gas adsorption canister increased based on the reference weighing value as the weight data.

In the method, after determining whether to trigger an alarm based on the weight data and at least one preset alarm threshold, the method further includes:
upon determining to trigger an alarm, sending an alarm instruction to the weigher such that the weigher gives an alarm upon receiving the alarm instruction.

An embodiment of the disclosure provides a medical alarm method, including:
displaying a preset weight setting control on a preset anesthesia interface;
upon receiving a trigger operation for the preset weight setting control, a weight setting panel popping up on the preset anesthesia interface in response to the trigger operation;
displaying at least one numerical value input control on the weight setting panel, and receiving input data by means of the at least one numerical value input control;
determining a first preset weight threshold based on the input data;
receiving weight data acquired and sent by a weigher in real time; and
determining to trigger an alarm and outputting a first alarm prompt if the weight data is greater than the first preset weight threshold, wherein the first alarm prompt includes at least one of sound, flashing light and an interface prompt.

An embodiment of the disclosure provides a medical alarm method, including:
acquiring weight data of a waste gas adsorption canister by weighing in real time, and transmitting the weight data to an anesthesia device, such that the anesthesia device determines whether to trigger an alarm based on the weight data and at least one preset alarm threshold, and outputs an alarm prompt when it is determined to trigger an alarm, wherein the at least one preset alarm threshold is a data criterion for an adsorption capacity abnormality of the waste gas adsorption canister or a data abnormality of a weigher; and the alarm prompt includes at least one of sound, flashing light and an interface prompt.

The above-mentioned method further includes:
upon receiving a weighing initialization instruction, taking the currently acquired weight data as a reference weighing value, so as to take a weight of the waste gas adsorption canister increased based on the reference weighing value as the weight data, wherein the weighing initialization instruction is an instruction that is sent by the anesthesia device to the weigher by means of the preset anesthesia interface, in response to an initialization trigger operation, upon receiving the initialization trigger operation for the preset initialization control.

In the above-mentioned method, after transmitting the weight data to the anesthesia device, the method further includes:
giving an alarm upon receiving an alarm instruction, wherein the alarm instruction is an instruction sent by the anesthesia device to the weigher when it is determined to trigger an alarm.

An embodiment of the disclosure provides a medical alarm method, including:
acquiring weight data of a waste gas adsorption canister by weighing in real time;
performing processing and analysis based on the weight data and at least one preset alarm threshold to obtain an analysis result; and
sending the analysis result to an anesthesia device, such that the anesthesia device determines whether to trigger an alarm based on the analysis result, and outputs an alarm prompt when it is determined to trigger an alarm; wherein the at least one preset alarm threshold is a data criterion for an adsorption capacity abnormality of the waste gas adsorption canister or a data abnormality of a weigher; and the alarm prompt includes at least one of sound, flashing light and an interface prompt.

In the above-mentioned method, the at least one preset alarm threshold includes a first preset weight threshold; and performing processing and analysis based on the weight data and at least one preset alarm threshold to obtain an analysis result includes:
taking the adsorption canister being overweight as the analysis result, if the weight data is greater than the first preset weight threshold.

In the above-mentioned method, the weigher is configured with a weight setting apparatus thereon; and before performing processing and analysis based on the weight data and at least one preset alarm threshold to obtain an analysis result, the method further includes:
receiving the first preset weight threshold by means of a weight setting apparatus.

In the above-mentioned method, the at least one preset alarm threshold includes a second preset weight threshold; and performing processing and analysis based on the weight data and at least one preset alarm threshold to obtain an analysis result includes:
upon receiving a ventilation initiation instruction, taking the currently acquired weight data as startup weight data to determine whether the startup weight data exceeds the second preset weight threshold, wherein the ventilation initiation instruction is an instruction sent by the anesthesia device to the weigher each time ventilation is initiated, and the second preset weight threshold is smaller than the first preset weight threshold; and
taking an available usage amount of the adsorption canister being insufficient as the analysis result, if the startup weight data is greater than the second preset weight threshold.

In the above-mentioned method, the at least one preset alarm threshold includes a preset slope threshold; and performing processing and analysis based on the weight data and at least one preset alarm threshold to obtain an analysis result includes:
acquiring a current ventilation parameter from the anesthesia device in real time;
recording corresponding time information of acquiring the weight data;
generating a slope of weight increase of the waste gas adsorption canister based on the weight data and the time information, wherein the slope of weight increase represents a historical trend of weight increase of the waste gas adsorption canister; and
taking the adsorption canister being overweight as the analysis result if the current ventilation parameter is not changed and the slope of weight increase is smaller than the preset slope threshold within a preset time period.

In the above-mentioned method, after generating a slope of weight increase of the waste gas adsorption canister based on the weight data and the time information, the method further includes:
obtaining a residual usage amount of the waste gas adsorption canister based on the first preset weight threshold and the current ventilation parameter, and sending the residual usage amount to the anesthesia device so as to display the residual usage amount by means of the anesthesia device in real time.

In the above-mentioned method, the at least one preset alarm threshold includes a preset weight range; and after acquiring weight data of the waste gas adsorption canister in real time, the method further includes:
determining that the analysis result is that the weigher is faulty, if the acquired weight data exceeds the preset weight range.

In the above-mentioned method, an information input control is preset on the weight setting apparatus; receiving the first preset weight threshold by means of a weight setting apparatus includes:
receiving target specification information by means of the information input control; and determining a preset weight threshold corresponding to the target specification information as the first preset weight threshold according to the target specification information and based on a preset correspondence between at least one term of preset specification information and at least one preset weight threshold.

In the above-mentioned method, the weigher is configured with a preset initialization button thereon, and the method further includes:
upon receiving a weighing initialization instruction sent by the anesthesia device, taking currently acquired weight data as a reference weighing value, so as to take a weight of the waste gas adsorption canister increased based on the reference weighing value as the weight data.

In the above-mentioned method, after sending the analysis result to the anesthesia device such that the anesthesia device determines whether to trigger an alarm based on the analysis result, the method further includes:
outputting an alarm prompt if the analysis result is any one of the adsorption canister being overweight, the available usage amount of the adsorption canister being insufficient and the weigher being faulty.

An embodiment of the disclosure provides a medical alarm method, including:
receiving an analysis result sent by a weigher, wherein the analysis result is obtained by acquiring weight data of a waste gas adsorption canister by the weigher in real time and performing processing and analysis based on the weight data and at least one preset alarm threshold, wherein the at least one preset alarm threshold is a data criterion for an adsorption capacity abnormality of the waste gas adsorption canister or a data abnormality of the weigher; and
determining whether to trigger an alarm based on the analysis result, and outputting an alarm prompt when it is determined to trigger an alarm, wherein the alarm prompt includes at least one of sound, flashing light and an interface prompt.

In the above-mentioned method, determining whether to trigger an alarm based on the analysis result and outputting an alarm prompt when it is determined to trigger an alarm include:
determining to trigger an alarm and output a first alarm prompt, if the analysis result is that the adsorption canister is overweight.

In the above-mentioned method, the at least one preset alarm threshold includes a second preset weight threshold; before receiving an analysis result sent by a weigher, the method further includes:
sending a ventilation initiation instruction to the weigher each time ventilation is initiated; and
determining whether to trigger an alarm based on the analysis result and outputting an alarm prompt when it is determined to trigger an alarm includes:
   determining to trigger an alarm and output a second alarm prompt if the analysis result is that an available usage amount of the adsorption canister is insufficient.

The above-mentioned method further includes:
receiving a residual usage amount of the waste gas adsorption canister sent by the weigher; and
displaying the residual usage amount in real time.

In the above-mentioned method, the at least one preset alarm threshold includes a preset weight range; before receiving an analysis result sent by a weigher, the method further includes:
determining to trigger an alarm and outputting a third alarm prompt if the analysis result is not received within a second preset time interval; or
after receiving the analysis result sent by the weigher, the method further includes:
   determining to trigger an alarm and output a third alarm prompt if the analysis result is that the weigher is faulty.

In the above-mentioned method, after determining whether to trigger an alarm based on the analysis result, the method further includes:
saving a current alarm event in a preset alarm record list upon triggering an alarm; and
upon receiving a preset view instruction, displaying the preset alarm record list in response to the preset view instruction.

An embodiment of the disclosure provides an anesthesia device, including: a respiratory branch, a driving device, a first communication apparatus, a first memory, a first processor and a first alarm apparatus; wherein
the driving device is configured to drive an anesthetic gas to the respiratory branch in an inspiratory phase;
the respiratory branch is configured to deliver the anesthetic gas to an anesthesia subject in the inspiratory phase; to receive an expiratory gas from the anesthesia subject, to obtain an unabsorbed waste anesthetic gas, and to deliver the expiratory gas and the waste anesthetic gas to the driving device in an expiratory phase;
the driving device is further configured to discharge the waste anesthetic gas and the expiratory gas to a waste gas adsorption canister such that the waste gas adsorption canister adsorbs the waste anesthetic gas and discharges the expiratory gas outside;
the first communication apparatus is configured to receive weight data of the waste gas adsorption canister acquired and sent by a weigher in real time;
the first memory is configured to store executable instructions; and
the first processor is configured to, when executing the executable instructions stored in the first memory, carry out the following steps:
   determining whether to trigger an alarm based on the weight data and at least one preset alarm threshold; and outputting an alarm prompt by means of the first alarm apparatus when it is determined to trigger an alarm, wherein the at least one preset alarm threshold is a data criterion for an adsorption capacity abnormality of the waste gas adsorption canister or a data abnormality of the weigher, and the alarm prompt includes at least one of sound, flashing light and an interface prompt.

An embodiment of the disclosure provides a weigher, including a weighing platform, a weighing body and a second communication apparatus, wherein
the weighing platform is configured to place a waste gas adsorption canister;
the weighing body is configured to acquire weight data of the waste gas adsorption canister in real time; and
the second communication apparatus is configured to transmit the weight data to an anesthesia device such that the anesthesia device determines whether to trigger an alarm based on the weight data and at least one preset alarm threshold, and outputs an alarm prompt when it is determined to trigger an alarm, wherein the at least one preset alarm threshold is a data criterion for an adsorption capacity abnormality of the waste gas adsorption canister or a data abnormality of the weigher.

An embodiment of the disclosure provides a first computer-readable storage medium that stores executable instructions, which are configured to, when executed by a first processor, implement one of the medical alarm methods provided in the embodiments of the disclosure.

An embodiment of the disclosure provides a second computer-readable storage medium that stores executable instructions, which are configured to, when executed by a second processor, implement one of the medical alarm methods provided in the embodiments of the disclosure.

In the medical alarm system according to the embodiments of the disclosure a weight of the waste gas adsorption canister measured on the weigher in real time can be synchronized to the anesthesia device by means of mutual communication between the weigher and the anesthesia device, and can further be used in combination with at least one preset alarm threshold to analyze usage status of the waste gas adsorption canister and determine whether the current operation status of the waste gas adsorption canister is an alarm scenario corresponding to the at least one preset alarm threshold, in which alarm scenario a use abnormality occurs, so as to determine to trigger an alarm or not. Accordingly, the interconnection and intercommunication between the weigher and the anesthesia device are achieved, such that abnormal data measured by the weigher can be integrated into the anesthesia device for unified monitoring and analysis, which provides a basis for further linkage processing with the weigher based on the analysis of the abnormal data and improves the intelligence of the medical alarm system. In addition, on the basis that the anesthesia device can be used to determine whether to trigger an alarm, it is further convenient to give a normalized alarm prompt using the anesthesia device, such that a user can acquire alarm prompt information more timely, thereby improving the safety of the medical alarm system.

### BRIEF DESCRIPTION OF THE DRAWINGS

FIG. 1 is a first structural diagram of an exemplary medical alarm system according to an embodiment of the disclosure;
FIG. 2 is a schematic design diagram of an exemplary preset anesthesia interface according to an embodiment of the disclosure;
FIG. 3 is a first flow chart of an exemplary medical alarm method according to an embodiment of the disclosure;
FIG. 4 is a second flow chart of an exemplary medical alarm method according to an embodiment of the disclosure;
FIG. 5 is a third flow chart of an exemplary medical alarm method according to an embodiment of the disclosure;
FIG. 6 is a fourth flow chart of an exemplary medical alarm method according to an embodiment of the disclosure;
FIG. 7 is a fifth flow chart of an exemplary medical alarm method according to an embodiment of the disclosure;
FIG. 8 is a sixth flow chart of an exemplary medical alarm method according to an embodiment of the disclosure;
FIG. 9 is a schematic structural diagram of an exemplary anesthesia device according to an embodiment of the disclosure;
FIG. 10 is a first schematic structural diagram of an exemplary weigher according to an embodiment of the disclosure; and
FIG. 11 is a second structural schematic diagram of an exemplary weigher according to an embodiment of the disclosure.

### DESCRIPTION OF THE PREFERRED EMBODIMENTS

The technical solutions in the embodiments of the disclosure will be clearly and completely described below in conjunction with the accompanying drawings in the embodiments of the disclosure.

In order to understand the features and technical contents of embodiments of the disclosure in more detail, the implementation of the embodiments of the disclosure will be described below in detail with reference to the accompanying drawings, which are for reference and illustration only, and are not intended to limit the embodiments of the disclosure.

At present, for anesthesia of a surgical subject in clinical practice, such as a surgery for a small animal in an animal hospital, the small animal is generally anesthetized by using a low-flow inhalation anesthesia method. Except inhaled anesthetics ingested by the animal, an excess gas is discharged from a waste gas discharge port of an anesthesia machine. If the waste gas is directly discharged outside an operation room by using a discharge tube, air pollution will be caused; and if it is directly discharged to the operation room, it will adversely affect the health of a user in the operation room, such as medical care personnel, so it is necessary to collect and discharge the waste gas generated in anesthesia. At present, a waste gas treatment solution commonly used in animal hospitals is to adsorb the waste gas containing an anesthetic and discharged from the anesthesia machine by using an activated carbon adsorption canister. With this solution, it is required to connect an inlet of the activated carbon adsorption canister to the waste gas discharge port of the anesthesia machine via a hose, the anesthetic (isoflurane, sevoflurane, etc.) in the waste gas is adsorbed by activated carbon, and clean air after filtration is discharged to the operation room through an outlet of the activated carbon adsorption canister. However, the activated carbon adsorption canister has an upper limit of adsorption capacity, and the weight of activated carbon particles will increase after the anesthetic is adsorbed. When the weight exceeds a preset specification, it is necessary to replace the activated carbon adsorption canister in time, otherwise there is risk of anesthetic leakage into the operation room. In order to monitor operating conditions of the adsorption canister and remind the user to replace the activated carbon adsorption canister in time, several solutions are provided as follows.

In a first solution, an anesthetic gas monitoring device is additionally provided at an outlet end of the activated carbon adsorption canister, and if it is detected that the concentration of the anesthetic gas exceeds a standard, the user is reminded to replace the activated carbon adsorption canister. But the anesthetic gas monitoring device is manufactured at an excessive cost and has few clinical applications.

In a second solution, the activated carbon adsorption canister is weighed by a weigher, and after the weight exceeds a preset specification, the weigher gives an alarm. This solution is widely used, but in actual use, the weigher cannot often provide a prompting mode of acoustic and optical alarms which meets standard specifications due to the limitations of its own hardware conditions; and furthermore, since the waste gas discharge port of the anesthesia machine is generally located on the back side of the machine, the waste gas adsorption canister and the weigher therefor are typically also placed close to the waste gas discharge port on the back side of the anesthesia machine. In complicated surroundings in the operation room, it is difficult to see alarm light on the back side of the anesthesia machine, and alarm sound is also likely to be ignored. If the user does not notice the alarm and replace the waste gas adsorption canister in time, the anesthetic gas may be directly discharged into the operation room, causing harm to the health of the user. In addition, since the preset specifications for the maximum weights of different brand types of waste gas adsorption canisters are different, it is necessary to set different alarm thresholds when different brand types of waste gas adsorption canisters are used. Existing weighers often have no screen or a small screen and have no button or few buttons such that if the user wishes to change or view a current alarm threshold, it is complicated to operate the weigher and is not easy to use the weigher in clinical practices. Therefore, the second solution also has relatively poor compatibility with different brand types of waste gas adsorption canisters.

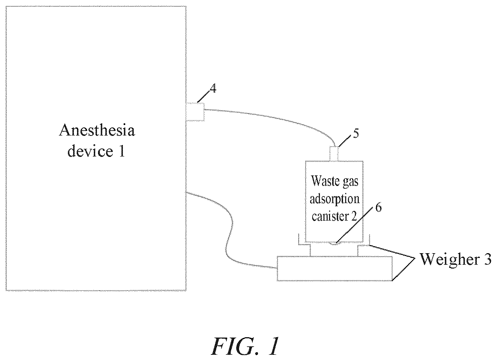

An embodiment of the disclosure provides a medical alarm system. As shown in FIG. 1, the medical alarm system is composed of an anesthesia device 1, a waste gas adsorption canister 2 and a weigher 3. A waste gas discharge port 4 of the anesthesia device 1 is connected to an inlet 5 of the waste gas adsorption canister 2 via a hose. The waste gas adsorption canister 2 is placed in a preset weight measurement region on the weigher 3, an outlet 6 of the waste gas adsorption canister 2 is directly exposed to the atmosphere, and the weigher 3 is in mutual communication with the anesthesia device 1 in a wired or wireless connection manner. The anesthesia device 1 is configured to release an anesthetic gas and to discharge a waste anesthetic gas; and the waste gas adsorption canister 2 is configured to adsorb the waste anesthetic gas.

During a surgery, a waste gas containing an anesthetic will be continually discharged through the waste anesthetic discharge port 4 of the anesthesia device 1, and when the waste gas passes through the waste gas adsorption canister 2, the anesthetic is adsorbed by an adsorbent, such as activated carbon, inside the waste gas adsorption canister 2, such that the weight of the waste gas adsorption canister 2 gradually increases. The medical alarm system provided in the embodiment of the disclosure can acquire weight data of the waste gas adsorption canister 2 by weighing the waste gas adsorption canister 2 by the weigher 3 in real time. Specifically, the weigher 3 can directly weigh the waste gas adsorption canister 2, and acquire the total weight of the waste gas adsorption canister 2 in real time as weight data; and the weigher 3 may also reset the currently acquired weight of the waste gas adsorption canister upon receiving a reset instruction, and take a weight increase of the waste gas adsorption canister 2 acquired in real time after the resetting as the weight data.

In an embodiment of the disclosure, the anesthesia device is configured with a first alarm apparatus. Based on the system structure shown in FIG. 1, in a medical alarm system provided by the disclosure, the weight data can be regularly transmitted to the anesthesia device by the weigher, and current usage status of the waste gas adsorption canister is analyzed by the anesthesia device based on the acquired weight data and at least one preset alarm threshold to determine whether to trigger an alarm or not. When it is determined to trigger an alarm, the anesthesia device outputs an alarm prompt by means of the first alarm apparatus, wherein the alarm prompt includes at least one of sound, flashing light and an interface prompt.

In the above-mentioned embodiment, the at least one preset alarm threshold is a data criterion for an adsorption capacity abnormality of the waste gas adsorption canister or a data abnormality of the weigher. In some embodiments, the at least one preset alarm threshold includes a first preset weight threshold. If the weight data is greater than the first preset weight threshold, it is indicated that the waste gas adsorption canister has reached a state of adsorption saturation; and the anesthesia device determines to trigger an alarm and outputs a first alarm prompt. The first alarm prompt is an alarm prompt given in a first preset mode.

In the above-mentioned embodiment, an acoustic-optical element and a display screen are generally pre-configured on the anesthesia device. When the anesthesia device outputs the first alarm prompt, sound and/or flashing light corresponding to the first alarm prompt may be outputted by the acoustic-optical element, and a text and/or an image corresponding to the first alarm prompt may be displayed on an interface of the display screen, so as to remind a user that the adsorption capacity of the waste gas adsorption canister reaches an upper limit and that timely replacement is needed. In some embodiments, the anesthesia device 1 may output an acoustic-optical alarm in the first mode according to the requirements of the standard 60601-1-8, and simultaneously display a preset alarm text and image on the display screen of the anesthesia device 1, and may also set a prompt mode of the first alarm prompt by itself according to the requirements of practical applications, and the embodiment of the disclosure is not limited thereto.

It will be appreciated that in the embodiment of the disclosure, the weigher and the anesthesia device may together form a complete alarm system by means of mutual communication between the weigher and the anesthesia device. Therefore, the weight of the waste gas adsorption canister acquired in real time on the weigher may be synchronized to the anesthesia device, and is then used to analyze the usage status of the waste gas adsorption canister in combination with at least one preset alarm threshold by the anesthesia device, so as to determine whether to trigger an alarm, thereby achieving the interconnection and intercommunication between the weigher and the anesthesia device, such that abnormal data measured by the weigher can be integrated into the anesthesia device for unified monitoring and analysis, which provides a basis for further linkage processing with the weigher based on the analysis of the abnormal data and improves the intelligence of the medical alarm system. In addition, a normalized alarm prompt by the anesthesia device is achieved, such that the user can acquire alarm prompt information more timely, thereby improving the safety of the medical alarm system. Furthermore, in the embodiment of the disclosure, the analysis, processing and alert of the weight data are all implemented on the anesthesia device, and the weigher is only responsible for the functions of data acquisition and transmission, thereby reducing the requirements for the size and weight of the weigher and improving system compatibility.

In the above-mentioned embodiment, the anesthesia device may be further configured with a human-machine interaction device, and a user's setting of the at least one alarm threshold is received by means of the human-machine interaction device. The human-machine interaction device may receive input data of the user by means of a variety of input means, such as a touchable display screen, a rotary encoder, or a button.

In some embodiments, when a display of the human-machine interaction device is a touchable display screen, the human-machine interaction device may display a preset weight setting control on a preset anesthesia interface by means of the display, and receive the input data by means of the preset weight setting control. In this way, the anesthesia device may determine the first preset weight threshold based on the input data.

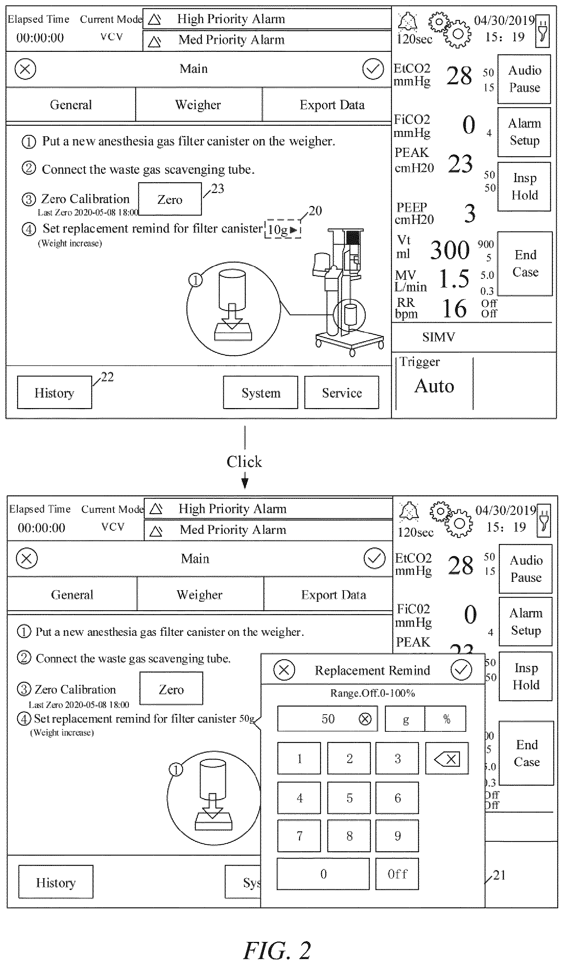

In some embodiments, the preset anesthesia interface may be as shown in FIG. 2, and the anesthesia device may exhibit a connection method of the medical alarm system and at least one menu item control for setting corresponding functions of the medical alarm system by means of the preset anesthesia interface. The preset weight setting control 20 is displayed on the preset anesthesia interface, the preset weight setting control 20 may be configured to exhibit the currently set first preset weight threshold, and when it is triggered by a preset operation event, such as a click event, a threshold setting panel 21 pops up, and the input data is received by the threshold setting panel 21. As an example, if preset specification information of the waste gas adsorption canister reveals that the maximum weight increase thereof is 50 g, the user may, after resetting an initial weight of the waste gas adsorption canister on the weigher before use, input 50 on the threshold setting panel and click a control OK, such that the anesthesia device can use the received input data of 50 g as the first preset weight threshold.

In some embodiments, the preset weight control on the preset anesthesia interface may also be a selection input control; and correspondence between at least one term of preset specification information of the waste gas adsorption canister and at least one preset weight threshold is pre-stored in the anesthesia device, and the human-machine interaction device may receive target specification information inputted by the user by means of the selection input control. In this way, the anesthesia device may determine a preset weight threshold corresponding to the target specification information as the first preset weight threshold based on the target specification information and the preset correspondence between the at least one term of preset specification information and the at least one preset weight threshold. As an example, the user may correspondingly preset different alarm thresholds of different brands of waste gas adsorption canisters in the anesthesia device, such that when a different brand of waste gas adsorption canister is used, a corresponding preset weight threshold can be automatically matched by directly selecting a pre-stored brand, which further improves the intelligence of the medical alarm system.

It can be understood that, in the embodiment of the disclosure, the medical alarm system may allow the user to both view and set the alarm threshold on the operation interface of the anesthesia device when using the anesthesia device for anesthetization, may further automatically match the alarm threshold according to different preset specification information, thereby improving the operability and intelligence of the medical alarm system.

In the above-mentioned embodiment, the medical alarm system may also detect a residual usage amount of the waste gas adsorption canister before a surgery, and remind the user of timely replacement when the residual usage amount of the waste gas adsorption canister is insufficient to complete the current surgery, so as to avoid interference with the surgery due to the replacement of the waste gas adsorption canister during the surgery. The at least one preset alarm threshold includes a second preset weight threshold , which is smaller than the first preset weight threshold. In some embodiments, the second preset weight threshold may be determined according to the preset specification information of the waste gas adsorption canister in conjunction with current estimated usage amount. As an example, the anesthesia device may, before initiating ventilation, estimate in advance the weight increase of the waste gas adsorption canister caused throughout current anesthesia as the current estimated usage amount according to currently set ventilation parameters, such as tidal volume and estimated duration of ventilation, and use a difference between the preset specification information of the waste gas adsorption canister and the current estimated usage amount as the second preset weight threshold. For example, if the anesthesia device estimates that the waste gas generated throughout the current anesthesia will increase the weight of the waste gas adsorption canister by 20 g, namely, the current estimated usage amount is 20 g, and the preset specification information of the waste gas adsorption canister is the maximum weight increase of 50 g, then the anesthesia device may set the second preset weight threshold at 30 g.

In the embodiment of the disclosure, the anesthesia device is further configured to take currently received weight data as startup weight data for each time ventilation is initiated, to determine whether the startup weight data exceeds the second preset weight threshold. If the startup weight data is greater than the second preset weight threshold, it is indicated that the residual usage amount of the waste gas adsorption canister is insufficient to complete the current surgery, and the anesthesia device determines to trigger an alarm and outputs a second alarm prompt. The second alarm prompt may be the same as or different from the first alarm prompt, and if the second alarm prompt is different from the first alarm prompt, a differentiated prompt may be given to the user so as to remind the user that the current available usage amount of the waste gas adsorption canister is insufficient.

In some embodiments, the second preset weight threshold is 30 g. If the startup weight data is 40 g, it is indicated that the startup weight data is greater than the second preset weight threshold, the waste gas adsorption canister will reach a saturation state by additionally increasing its weight by 10 g; if replacement is not carried out in advance, the surgery being performed may be affected after this surgery begins; therefore, the anesthesia device determines to trigger an alarm and outputs the second alarm prompt.

In the above-mentioned embodiment, the at least one preset alarm threshold includes a preset slope threshold; and
the anesthesia device is further configured to: record corresponding time information of acquiring the weight data; generate a slope of weight increase of the waste gas adsorption canister based on the weight data and the time information, wherein the slope of weight increase represents the trend of weight increase of the waste gas adsorption canister; acquire a current ventilation parameter in real time; and determine whether to trigger an alarm and output the first alarm prompt if the current ventilation parameter is not changed and the slope of weight increase is smaller than a preset slope threshold within a preset time period.

In the embodiment of the disclosure, since the weigher regularly sends the weight data acquired in real time to the anesthesia device, the anesthesia device may, when acquiring the weight data each time, correspondingly record the time information corresponding to the acquired weight data, so as to obtain a growth curve of the weight data based on the correspondence between the weight data and each term of time information on a time axis. The anesthesia device can obtain a slope of weight increase of the waste gas adsorption canister by filtering and averaging the growth curve of the weight data and calculating the slope of the curve, wherein the slope of weight increase represents the trend of weight increase of the waste gas adsorption canister.

In the embodiment of the disclosure, the anesthesia device may monitor the usage status of the waste gas adsorption canister according to the slope of weight increase. Specifically, the anesthesia device may acquire the current ventilation parameter in real time; and if the current ventilation parameter is not changed within the preset time period, it is indicated that the amount of gas currently inputted into the waste gas adsorption canister is not changed; however, in this case, if the slope of weight increase of the waste gas adsorption canister is smaller than a preset slope threshold, it is indicated that the trend of weight increase of the waste gas adsorption canister abnormally descends, which may result from incorrect reported weight data due to a failure of the weigher or a connection failure, and in this case, the anesthesia device determines to trigger an alarm and outputs the first alarm prompt.

It can be understood that in the embodiment of the disclosure, the anesthesia device can also give an alarm of adsorption saturation of the waste gas adsorption canister by monitoring the slope of weight increase, and even when there is a deviation in the accuracy of the weigher, the anesthesia device can still intelligently identify and determine whether the waste gas adsorption canister reaches the saturation state, which eliminates the risk of failing to perform correct monitoring when there is a problem with the acquired weight data, and provides dual protection for safety monitoring, thereby improving the safety of the medical alarm system.

In the above-mentioned embodiment, the anesthesia device is further configured to obtain a residual usage amount of the waste gas adsorption canister based on the first preset weight threshold and the current ventilation parameter, and to display the residual usage amount in real time by means of the display.

In the embodiment of the disclosure, the anesthesia device may further estimate the residual available usage amount of the waste gas adsorption canister based on the acquired current ventilation parameter in combination with the first preset weight threshold, and display the residual usage amount to the user by means of the display in real time during the use of the waste gas adsorption canister.

In some embodiments, the residual usage amount may be displayed in the form of residual available time, and may also be displayed graphically, such as in a progress bar, which may be selected specifically according to actual situations and is not limited in the embodiment of the disclosure.

It will be appreciated that in the embodiment of the disclosure, real-time display of the residual usage amount of the waste gas adsorption canister on the anesthesia device can better prompt the user of real-time usage status of the waste gas adsorption canister, which improves the usability of the medical alarm system.

In the above-mentioned embodiment, the anesthesia device is further configured to determine to trigger an alarm and output a third alarm prompt if the weight data transmitted by the weigher is not received within a first preset time interval or the received weight data exceeds the preset weight range, wherein the third alarm prompt is used to prompt that the weigher is faulty.

In the embodiment of the disclosure, the anesthesia device may also achieve state detection of the weigher by means of the mutual communication with the weigher. Specifically, the weigher will normally send the real-time acquired weight data regularly to the anesthesia device. If the anesthesia device does not receive the weight data transmitted by the weigher within the first preset time interval, it is indicated that there is a problem with the weigher itself or with a connection line between the weigher and the anesthesia device, and the anesthesia device may determine to trigger an alarm and output the third alarm prompt. Alternatively, if the weight data received by the anesthesia device exceeds the preset weight range, it is indicated that the weigher has a significant deviation in its accuracy and cannot operate normally, and the anesthesia device may determine to trigger an alarm and output the third alarm prompt.

It will be appreciated that in the embodiment of the disclosure, the anesthesia device may monitor abnormal operation status of the weigher and give an alarm in time, thereby further improving the safety of the medical alarm system.

In the above-mentioned embodiment, when it is monitored that the weight data of the waste gas adsorption canister exceeds the first preset weight threshold, the anesthesia device may also take a variety of intelligent linkage measures to further improve the usability and safety of the medical alarm system. As an example, the anesthesia device may actively lower the concentration of the anesthetic gas, or replace it with a more appropriate ventilation parameter or the like, which is specifically selected according to actual situations and is not limited in the embodiment of the disclosure.

In the above-mentioned embodiment, the anesthesia device is further configured to, upon triggering an alarm, save a current alarm event in a preset alarm record list; and the display is further configured to, upon receiving a preset view instruction, display the preset alarm record list in response to the preset view instruction.

In the embodiment of the disclosure, the anesthesia device may record alarm events of the waste gas adsorption canister to facilitate subsequent tracing of the waste gas adsorption canister. Specifically, when the anesthesia device determines to trigger an alarm based on the weight data and the at least one preset alarm threshold, the anesthesia device may save a current alarm event in the preset alarm record list, wherein the current alarm event may include, for example, information about events such as alarm time, current weight data, and which preset alarm threshold triggers the alarm. And a preset event viewing entry for displaying the preset alarm record list is provided on the preset anesthesia interface, such that upon receiving a preset view instruction for the preset event viewing entry by means of a display, the anesthesia device can display the preset alarm record list in response to the preset view instruction. In some embodiments, the preset event viewing entry may be a control 22 as illustrated in FIG. 2.

It will be appreciated that, in the embodiment of the disclosure, the medical alarm system may record and display historical alarm events to assist the user in counting various abnormalities arising during use of the waste gas adsorption canister, and thus improves the use of the waste gas adsorption canister, which improves the usability and safety of the medical alarm system.

In the above-mentioned embodiment, the display is further configured to display a preset initialization control on the preset anesthesia interface; the human-machine interaction device is further configured to, upon receiving an initialization trigger operation for the preset initialization control, send a weighing initialization instruction to the weigher in response to the initialization trigger operation; and the weigher is further configured to, upon receiving the weighing initialization instruction, take currently acquired weight data as a reference weighing value, so as to take a weight of the waste gas adsorption canister increased based on the reference weighing value as the weight data.

In the embodiment of the disclosure, the medical alarm system may also initialize a default reference weighing value of the weigher by using the human-machine interaction device configured on the anesthesia device, such as a weight resetting operation. Specifically, the display in the human-machine interaction device on the anesthesia device may display the preset initialization control, such as a resetting button, on the preset anesthesia interface, and upon receiving the initialization trigger operation for the preset initialization control, the human-machine interaction device sends the weighing initialization instruction to the weigher in response to the initialization trigger operation. The weigher, upon receiving the weighing initialization instruction, records currently acquired weight data as the reference weighing value, for example, records the currently acquired weight data as zero, such that the weigher can take the weight of the subsequently weighed waste gas adsorption canister increased based on the reference weighing value as the weight data. In some embodiments, the preset initialization control may be a control 23 as illustrated in FIG. 2.

In the above-mentioned embodiment, the weigher is configured with a second alarm apparatus; the anesthesia device is further configured to send an alarm instruction to the weigher upon determining to trigger an alarm. In this way, the weigher may give an alarm by means of the second alarm apparatus upon receiving the alarm instruction.

In the embodiment of the disclosure, an alarm apparatus may also be configured on the weigher in order to improve a prompt effect of an alarm. In this way, when the anesthesia device determines to trigger an alarm according to the current usage status of the waste gas adsorption canister, the anesthesia device may synchronously send the alarm instruction to the weigher such that the weigher, upon receiving the alarm instruction, gives an alarm synchronously with the anesthesia device by means of its own alarm apparatus.

In another embodiment of the disclosure, the anesthesia device is configured with a first alarm apparatus. Based on the system structure shown in FIG. 1, another medical alarm system provided in the disclosure may acquire the weight data of the waste gas adsorption canister in real time by the weigher, perform processing and analysis based on the weight data and the at least one preset alarm threshold to obtain an analysis result, and then send the analysis result to the anesthesia device such that the anesthesia device determines whether to trigger an alarm based on the analysis result; and when it is determined to trigger an alarm, the anesthesia device may output an alarm prompt by means of the first alarm apparatus, wherein the alarm prompt includes at least one of sound, flashing light and an interface prompt.

In the embodiment of the disclosure, the medical alarm system may also realize processing and analysis of the weight data by means of the weigher, wherein the analysis result indicates whether an adsorption capacity of the waste gas adsorption canister is abnormal or whether the weigher has abnormal data. Th process for the weigher to perform processing and analysis based on the weight data and the at least one preset alarm threshold to obtain the analysis result is similar to the process on the anesthesia device, which will not be described in detail herein. The weigher can regularly send the analysis result indicating normality or abnormality to the anesthesia device, and the anesthesia device determines whether to give an alarm according to the analysis result and correspondingly outputs an alarm prompt.

In the above-mentioned embodiment, the at least one preset alarm threshold includes a first preset weight threshold; the weigher is further configured to send the analysis result indicating that the adsorption canister is overweight, to the anesthesia device, if the weight data is greater than the first preset weight threshold; and the anesthesia device is further configured to determine to trigger an alarm and output a first alarm prompt if the analysis result is that the canister is overweight.

In the embodiment of the disclosure, when detecting that the weight data is greater than the first preset weight threshold, the weigher sends the analysis result indicating that the adsorption canister is overweight, to the anesthesia device, and the anesthesia device determines to trigger an alarm and output the first alarm prompt based on the analysis result of the adsorption canister being overweight.

In the above-mentioned embodiment, the weigher is configured with a weight setting apparatus thereon which is configured to receive the first preset weight threshold.

In the above-mentioned embodiment, an information input control is preset on the weight setting apparatus; the information input control is configured to receive target specification information; and the weigher is further configured to determine a preset weight threshold corresponding to the target specification information as the first preset weight threshold according to the target specification information and a preset correspondence between at least one term of preset specification information and at least one preset weight threshold.

In the embodiment of the disclosure, the medical alarm system may implement the setting of the first preset weight threshold by means of the weight setting apparatus configured on the weigher, such as a button, a knob, or a touch display screen. The process therefor is similar to the process for the anesthesia device to determine the first preset weight threshold, and will not be described in detail herein.

In the above-mentioned embodiment, the at least one preset alarm threshold includes a second preset weight threshold; the anesthesia device is further configured to send a ventilation initiation instruction to the weigher each time ventilation is initiated; the weigher is further configured to, upon receiving the ventilation initiation instruction, take the currently acquired weight data as startup weight data, to determine whether the startup weight data exceeds the second preset weight threshold, and second the analysis result indicating that an available usage amount of the adsorption canister is insufficient, to the anesthesia device, if the startup weight data is greater than the second preset weight threshold, wherein the second preset weight threshold is smaller than the first preset weight threshold; and the anesthesia device is further configured to determine to trigger an alarm and output a second alarm prompt if the analysis result is that the available usage amount of the adsorption canister is insufficient..

In the embodiment of the disclosure, with the weight data being analyzed by the weigher, the anesthesia device may send the ventilation initiation instruction to the weigher each time the ventilation is initiated, and the weigher, upon receiving the ventilation initiation instruction, obtains the analysis result based on the startup weight data and the second preset weight threshold, and the anesthesia device then determines whether to trigger an alarm of the second alarm prompt according to the analysis result.

In the above-mentioned embodiment, the at least one preset alarm threshold includes a preset slope threshold; wherein the weigher is further configured to acquire a current ventilation parameter from the anesthesia device in real time; to record corresponding time information of acquiring the weight data; to generate a slope of weight increase of the waste gas adsorption canister based on the weight data and the time information, wherein the slope of weight increase represents a historical trend of weight increase of the waste gas adsorption canister; and to send the analysis result indicating that the adsorption canister is overweight, to the anesthesia device, if the current ventilation parameter is not changed and the slope of weight increase is smaller than the preset slope threshold within a preset time period; and the anesthesia device is further configured to determine to trigger an alarm and output the first alarm prompt if the analysis result is that the canister is overweight.

In the embodiment of the disclosure, the weigher may also determine whether the waste gas adsorption canister is overweight by monitoring and analyzing the slope of weight increase; and the process for the monitoring and analysis is similar to the implementation process for the anesthesia device and will not be described in detail herein.

In the above-mentioned embodiment, the anesthesia device is configured with a display, and the weigher is further configured to obtain a residual usage amount of the waste gas adsorption canister based on the first preset weight threshold and the current ventilation parameter, and to send the residual usage amount to the anesthesia device; and the anesthesia devices is further configured to display the residual usage amount in real time by means of the display.

In the embodiment of the disclosure, the weigher may also obtain the residual usage amount of the waste gas adsorption canister based on the analysis and estimation on the first preset weight threshold and the current ventilation parameter, and the process therefor is similar to the implementation process for the above-described anesthesia device and will not be described in detail herein. The weigher may send the residual usage amount to the anesthesia device, and display the residual usage amount to the user by using the display configured on the anesthesia device.

In the above-mentioned embodiment, the at least one preset alarm threshold includes a preset weight range; wherein
the weigher is further configured to determine that the analysis result is that the weigher is faulty, if the acquired weight data exceeds the preset weight range; and the anesthesia device is further configured to trigger an alarm and output a third alarm prompt, if the analysis result is not received within a second preset time interval or the analysis result is that the weigher is faulty.

In the embodiment of the disclosure, since the weigher will normally send the analysis result regularly to the anesthesia device, when the anesthesia device does not receive the analysis result within the second preset time interval, there may be a fault in the weigher itself or in a connection between the anesthesia device and the weigher. In this case, the anesthesia device may determine to trigger an alarm and output the third alarm prompt. In addition, the weigher may also perform a built-in check on the weight data acquired by itself, and when the weight data exceeds the preset weight range, the weigher determines that the analysis result is that the weigher is faulty, and sends the analysis result to the anesthesia device, and the anesthesia device determines to trigger the corresponding third alarm prompt.

In the above-mentioned embodiment, the anesthesia device is further configured to, upon triggering an alarm, save a current alarm event in a preset alarm record list; and the display is further configured to, upon receiving a preset view instruction, display the preset alarm record list in response to the preset view instruction.

In the above-mentioned embodiment, the weigher is further configured with a preset initialization button, wherein the weigher is further configured to, upon receiving a trigger operation for the preset initialization button, take currently acquired weight data as a reference weighing value in response to the trigger operation, so as to take a weight of the waste gas adsorption canister increased based on the reference weighing value as the weight data.

In the embodiment of the disclosure, the weigher may also implement a calibration process for the reference weighing value by means of the preset initialization button configured thereon.

In the above-mentioned embodiment, the weigher is configured with a second alarm apparatus, wherein the weigher is configured to give an alarm by means of the second alarm apparatus when the analysis result is any one of the adsorption canister being overweight, the available usage amount of the adsorption canister being insufficient and the weigher being faulty.

In the embodiment of the disclosure, the weigher may also directly give an alarm by means of its own alarm apparatus in any abnormal situation of the adsorption canister being overweight, the available usage amount of the adsorption canister being insufficient and the weigher being faulty.

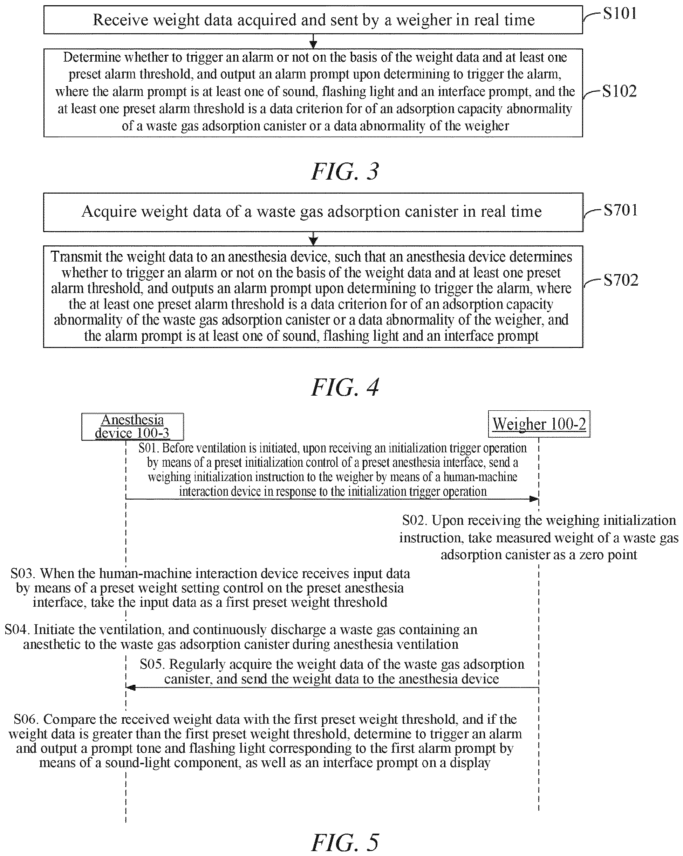

An embodiment of the disclosure provides a medical alarm method. Steps of the method are described below with reference to FIG. 3 with application of the method to an anesthesia device as an example.

At step S101, weight data acquired and sent by a weigher in real time is received.

At step S102, whether to trigger an alarm is determined based on the weight data and at least one preset alarm threshold, and an alarm prompt is outputted when it is determined to trigger an alarm, wherein the alarm prompt includes at least one of sound, flashing light and an interface prompt; and the at least one preset alarm threshold is a data criterion for an adsorption capacity abnormality of a waste gas adsorption canister or a data abnormality of the weigher.

In the embodiment of the disclosure, the anesthesia device is configured with a first alarm apparatus thereon, and upon determining to trigger an alarm, the anesthesia device may output the alarm prompt by means of the first alarm apparatus.

In some embodiments, the at least one preset alarm threshold includes a first preset weight threshold; and step S102 may also include step S1021 as follows:
at step S1021, it is determined to trigger an alarm and output a first alarm prompt if the weight data is greater than the first preset weight threshold.

In some embodiments, the anesthesia device is configured with a human-machine interaction device thereon which includes a display; and prior to step S102, steps S201-S202 may further be included as follows.

At step S201, a preset input control is displayed on a preset anesthesia interface by means of the display, and input data is inputted by means of the preset input control.

At step S202, the first preset weight threshold is determined based on the input data.

In some embodiments, the preset input control may be a preset weight setting control, and step S201 may include: displaying the preset weight setting control on the preset anesthesia interface, and upon receiving a trigger operation for the preset weight setting control, a weight setting panel popping up on the preset anesthesia interface in response to the trigger operation; displaying at least one numerical value input control on the weight setting panel, and receiving the input data by means of the at least one numerical value input control; and determining the first preset weight threshold based on the input data.

Here, a process for the anesthesia device to determine the first preset weight threshold by means of the preset anesthesia interface is similar to that shown in FIG. 2, and will not be described in detail herein.

In some embodiments, the preset weight setting control may be a selection input control, and step S201 may include step S2011 as follows.

At step S2011, target specification information is received as the input data by means of the selection input control; and
accordingly, step S202 may include step S2021 as follows.

At step S2021, if the input data is the target specification information, a preset weight threshold corresponding to the target specification information is determined as the first preset weight threshold according to a preset correspondence between at least one term of preset specification information and at least one preset weight threshold.

In some embodiments, the at least one preset alarm threshold includes a second preset weight threshold; and step S102 may include steps S1022-S1023 as follows.

At step S1022, each time ventilation is initiated, currently received weight data is taken as startup weight data to determine whether the startup weight data exceeds the second preset weight threshold, wherein the second preset weight threshold is smaller than the first preset weight threshold.

At step S1023, it is determined to trigger an alarm and output a second alarm prompt if the startup weight data is greater than the second preset weight threshold.

In some embodiments, the at least one preset alarm threshold includes a preset slope threshold; and step S102 may include steps S1024-S1027 as follows.

At step S1024, time information corresponding to the weight data is recorded.

At step S1025, a slope of weight increase of the waste gas adsorption canister is generated based on the weight data and the time information, wherein the slope of weight increase represents a historical trend of weight increase of the waste gas adsorption canister.

At step S1026, a current ventilation parameter is acquired in real time.

At step S1027, it is determined to trigger an alarm and output the first alarm prompt if a current ventilation parameter is not changed and the slope of weight increase is smaller than the preset slope threshold within a preset time period.

In some embodiments, posterior to step S1026, steps 5301-5302 may further be included as follows.

At step S301, a residual usage amount of the waste gas adsorption canister is obtained based on the first preset weight threshold and the current ventilation parameter.

At step S302, the residual usage amount is displayed in real time by means of the display.

In some embodiments, the at least one preset alarm threshold includes a preset weight range; and prior to step S101, step S401 may further be included as follows.

At step S401, it is determined to trigger an alarm and output a third alarm prompt if the weight data is not received within a preset time interval, wherein the third alarm prompt represents that the weigher is faulty.

Alternatively, posterior to step S101, step S402 may further be included as follows.

At step S402, it is determined to trigger an alarm and output a third alarm prompt if the weight data exceeds a preset numerical value range.

It should be noted that in the embodiment of the disclosure, step S1021, steps S1022-S1023, steps S1024-S1027 and step S402 are parallel method processes corresponding to different preset alarm thresholds, and during particular implementation, a corresponding method process may be correspondingly performed according to the weight data or a numerical value of the slope of weight increase.

In some embodiments, posterior to step S102, steps S103-S104 may further be included as follows.

At step S103, a current alarm event is saved in a preset alarm record list when an alarm is triggered.

At step S104, upon receiving a preset view instruction, the preset alarm record list is displayed in response to the preset view instruction.

In some embodiments, the medical alarm method provided in the embodiment of the disclosure may further include steps S501-S502 as follows.

At step S501, a preset initialization control is displayed on the preset anesthesia interface.

At step S502, upon receiving an initialization trigger operation for the preset initialization control, a weighing initialization instruction is sent to the weigher in response to the initialization trigger operation, such that the weigher, upon receiving the weighing initialization instruction, takes currently acquired weight data as a reference weighing value, so as to take a weight of the waste gas adsorption canister increased based on the reference weighing value as the weight data.

In some embodiments, after determining whether to trigger an alarm based on the weight data and the at least one preset alarm threshold in step S102, step S601 may further be included as follows.

At step S601, upon determining to trigger an alarm, an alarm instruction is sent to the weigher such that the weigher gives an alarm upon receiving the alarm instruction.

In the embodiment of the disclosure, the weigher is configured with a second alarm apparatus thereon; and when receiving the alarm instruction sent by the anesthesia device, the weigher may give an alarm by means of the second alarm apparatus.

An embodiment of the disclosure provides a medical alarm method, which can be applied to a weigher of a medical alarm system. As shown in FIG. 4, the method may include steps S701-S702 as follows.

At step S701, weight data of a waste gas adsorption canister is acquired by weighing in real time.

At step S702, the weight data is transmitted to an anesthesia device, such that the anesthesia device determines whether to trigger an alarm based on the weight data and at least one preset alarm threshold, and outputs an alarm prompt upon determining to trigger an alarm, wherein the at least one preset alarm threshold is a data criterion for an adsorption capacity abnormality of the waste gas adsorption canister or a data abnormality of a weigher; and the alarm prompt includes at least one of sound, flashing light and an interface prompt.

In the embodiment of the disclosure, the anesthesia device is configured with a first alarm apparatus thereon, and upon determining to trigger an alarm, the anesthesia device may output the alarm prompt by means of the first alarm apparatus.

In some embodiments, the medical alarm method provided in the embodiment of the disclosure further includes:
upon receiving a weighing initialization instruction sent by the anesthesia device, taking currently acquired weight data as a reference weighing value, so as to take a weight of the waste gas adsorption canister increased based on the reference weighing value as the weight data.

In some embodiments, the weigher is configured with a second alarm apparatus thereon; after acquiring the weight data of the waste gas adsorption canister by weighing in real time and transmitting the weight data to the anesthesia device, the method further includes:
giving an alarm upon receiving an alarm instruction, wherein the alarm instruction is an instruction sent when the anesthesia device determines to trigger the alarm.

In the embodiment of the disclosure, the weigher may, upon receiving the alarm instruction sent by the anesthesia device, give the alarm by means of the second alarm apparatus configured thereon.

A typical exemplary application of an embodiment of the disclosure in a clinical application scenario will be described below with reference to FIG. 5. In a medical alarm system 100 of an embodiment of the disclosure, a brand-new waste gas adsorption canister is placed on a weigher 100-2, a waste gas discharge interface of an anesthesia device 100-3 is connected to an inlet of the waste gas adsorption canister via a hose, the anesthesia device 100-3 is connected to the weigher 100-2 in a wired manner, the anesthesia device 100-3 is configured with a human-machine interaction device which includes a display with a touch screen, and a preset anesthesia interface is displayed on the display. A workflow of the medical alarm system 100 in the embodiment of the disclosure will be described in conjunction with various steps.

At step S01, before the anesthesia device 100-3 initiates ventilation, upon receiving an initialization trigger operation by means of a preset initialization control of the preset anesthesia interface, a weighing initialization instruction is sent to the weigher 100-2 by means of the human-machine interaction device in response to the initialization trigger operation.

At step S02, the weigher 100-2, upon receiving the weighing initialization instruction, takes measured weight of the waste gas adsorption canister 100-1 as a zero point.

At step S03, when the human-machine interaction device of the anesthesia device 100-3 receives input data by means of a preset weight setting control on the preset anesthesia interface, the anesthesia device 100-3 takes the input data as a first preset weight threshold.

At step S04, the anesthesia device 100-3 initiates ventilation, and continuously discharges a waste gas containing an anesthetic to the waste gas adsorption canister during anesthesia ventilation.

At step S04, the anesthetic is adsorbed by activated carbon inside the waste gas adsorption canister, which resulting in an increase in the weight of the waste gas adsorption canister, and a gas after filtration by the activated carbon is discharged to the atmosphere through an outlet of the waste gas adsorption canister.

At step S05, the weigher 100-2 regularly acquires the weight data of the waste gas adsorption canister by weighing, and sends the weight data to the anesthesia device 100-3.

At step S06, the anesthesia device 100-3 compares the received weight data with the first preset weight threshold, and if the weight data is greater than the first preset weight threshold, the anesthesia device 100-3 determines to trigger an alarm and outputs a prompt tone and flashing light corresponding to the first alarm prompt by means of an acoustic-optical element, as well as an interface prompt on the display, so as to remind a user that the waste gas adsorption canister is full and needs to be replaced in time.

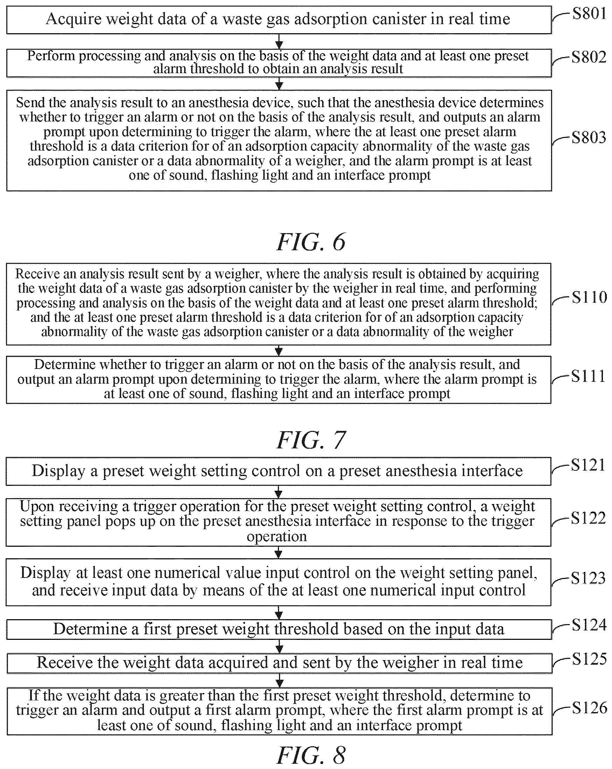

An embodiment of the disclosure provides a medical alarm method. Steps of the method are described below with reference to FIG. 6 with application of the method to a weigher as an example.

At step S801, weight data of a waste gas adsorption canister is acquired by weighing in real time.

At step S802, processing and analysis are performed based on the weight data and at least one preset alarm threshold to obtain an analysis result.

At step S803, the analysis result is sent to the anesthesia device, such that the anesthesia device determines whether to trigger an alarm based on the analysis result, and outputs an alarm prompt upon determining to trigger an alarm, wherein the at least one preset alarm threshold is a data criterion for an adsorption capacity abnormality of the waste gas adsorption canister or a data abnormality of the weigher; and the alarm prompt includes at least one of sound, flashing light and an interface prompt.

In the embodiment of the disclosure, the anesthesia device is configured with a first alarm apparatus thereon, and upon determining to trigger an alarm based on the analysis result, the anesthesia device may output an alarm prompt by means of the first alarm apparatus.

In some embodiments, the medical alarm method provided in the embodiment of the disclosure may further include: upon receiving a weighing initialization instruction sent by the anesthesia device, taking currently acquired weight data as a reference weighing value, so as to take a weight of the waste gas adsorption canister increased based on the reference weighing value as the weight data.

In some embodiments, the at least one preset alarm threshold includes a first preset weight threshold; and step S802 may include step S8021 as follows.

At step S8021, if the weight data is greater than the first preset weight threshold, the adsorption canister being overweight is taken as the analysis result.

In some embodiments, the weigher is configured with a weight setting apparatus thereon; and prior to step S802, step S901 may further be included as follows.

At step S901, the first preset weight threshold is received by means of the weight setting apparatus.

In some embodiments, the weigher may directly receive an externally inputted weight value as the first preset weight threshold by means of the weight setting apparatus.

In some embodiments, an information input control is preset on the weight setting apparatus; and the weigher may receive target specification information by means of the information input control, and determine a preset weight threshold corresponding to the target specification information as the first preset weight threshold according to a preset correspondence between at least one term of preset specification information and at least one preset weight threshold.

In some embodiments, the at least one preset alarm threshold includes a second preset weight threshold; and step S802 may include steps S8022-S8023 as follows.

At step S8022, upon receiving a ventilation initiation instruction, currently acquired weight data is taken as startup weight data to determine whether the startup weight data exceeds the second preset weight threshold, wherein the ventilation initiation instruction is an instruction sent by the anesthesia device to the weigher each time ventilation is initiated, and the second preset weight threshold is smaller than the first preset weight threshold.

At step S8023, if the startup weight data is greater than the second preset weight threshold, an available usage amount of the adsorption canister being insufficient is taken as the analysis result.

In some embodiments, the at least one preset alarm threshold includes a preset slope threshold; and step S702 may include steps S8024-S8027 as follows.

At step S8024, a current ventilation parameter is acquired from the anesthesia device in real time.

At step S8025, corresponding time information of acquiring the weight data is recorded.

At step S8026, a slope of weight increase of the waste gas adsorption canister is generated based on the weight data and the time information, wherein the slope of weight increase represents a historical trend of weight increase of the waste gas adsorption canister.

At step S8027, the adsorption canister being overweight is taken as the analysis result if the current ventilation parameter is not changed and the slope of weight increase is smaller than the preset slope threshold within a preset time period.

In some embodiments, posterior to step S8024, step S8024A may further be included as follows.

At step S8024A, a residual usage amount of the waste gas adsorption canister is obtained based on the first preset weight threshold and the current ventilation parameter, and the residual usage amount is sent to the anesthesia device so as to display the residual usage amount by means of the anesthesia device in real time.

In some embodiments, the at least one preset alarm threshold includes a preset weight range; and step S802 may further include step S8028 as follows.

At step S8028, if the acquired weight data exceeds the preset weight range, it is determined that the analysis result is that the weigher is faulty.

It should be noted that in the embodiment of the disclosure, step S8021, steps S8022-S8023, steps S8024-S8027 and step S8028 are parallel method processes corresponding to different preset alarm thresholds, and during particular implementation, a corresponding method process may be correspondingly performed according to the weight data or a numerical value of the slope of weight increase.

In some embodiments, the weigher is configured with a preset initialization button thereon. The medical alarm method provided in the embodiment of the disclosure further includes:
step S1001, upon receiving a trigger operation for the preset initialization button, currently acquired weight data is taken as a reference weighing value in response to the trigger operation, so as to take a weight of the waste gas adsorption canister increased based on the reference weighing value as the weight data.

In some embodiments, posterior to step S803, step S804 may further be included as follows.

At step S804, an alarm prompt is output if the analysis result is any one of the adsorption canister being overweight, the available usage amount of the adsorption canister being insufficient and the weigher being faulty.

In the embodiment of the disclosure, the weigher may be configured with a second alarm apparatus thereon, and if the analysis result of the weigher is any one of the adsorption canister being overweight, the available usage amount of the adsorption canister being insufficient, and the weigher being faulty, an alarm prompt is outputted by means of the second alarm apparatus.

An embodiment of the disclosure provides a medical alarm method as shown in FIG. 7, including steps S110-S111. The steps of the method are described below with application of the method to an anesthesia device of a medical alarm system as an example.

At step S110, an analysis result sent by a weigher is received, wherein the analysis result is obtained by acquiring weight data of a waste gas adsorption canister by a weigher in real time and performing processing and analysis based on the weight data and at least one preset alarm threshold, wherein the at least one preset alarm threshold is a data criterion for an adsorption capacity abnormality of the waste gas adsorption canister or a data abnormality of the weigher.

At step S 111, it is determined whether to trigger an alarm based on the analysis result and an alarm prompt is outputted upon determining to trigger an alarm, wherein the alarm prompt includes at least one of sound, flashing light and an interface prompt.

In the embodiment of the disclosure, the anesthesia device is configured with a first alarm apparatus thereon, and upon determining to trigger an alarm, the anesthesia device may output the alarm prompt by means of the first alarm apparatus.

In some embodiments, step S111 may include step S1111 as follows.

At step S1111, if the analysis result is that the adsorption canister is overweight, it is determined to trigger an alarm and output a first alarm prompt.

In some embodiments, the at least one preset alarm threshold includes a second preset weight threshold; and prior to step S110, the anesthesia device may send a ventilation initiation instruction to the weigher each time ventilation is initiated. In this way, the weigher may, upon receiving the ventilation initiation instruction, take currently acquired weight data as startup weight data to determine whether the startup weight data exceeds the second preset weight threshold; and if the startup weight data is greater than the second preset weight threshold, the analysis result indicating that an available usage amount of the adsorption canister is insufficient, is sent to the anesthesia device. Accordingly, step S111 may include step S1112 as follows.

At step S1112, if the analysis result is that the available usage amount of the adsorption canister is insufficient, it is determined to trigger an alarm and output a second alarm prompt.

In some embodiments, the anesthesia device is configured with a display, and the method further includes:
receiving a residual usage amount of the waste gas adsorption canister sent by the weigher; and
displaying the residual usage amount in real time.

In some embodiments, the at least one preset alarm threshold includes a preset weight range; and prior to step S110, the method may further include: determining to trigger an alarm and outputting a third alarm prompt if the analysis result is not received within a second preset time interval. Alternatively, posterior to step S110, the method may further include: if the analysis result is that the weigher is faulty, determining to trigger an alarm and output a third alarm prompt.

In some embodiments, posterior to step S111, steps S112-S113 may further be included as follows.

At step S112, a current alarm event is saved in a preset alarm record list when an alarm is triggered.

At step S113, upon receiving a preset view instruction, the preset alarm record list is displayed in response to the preset view instruction.

An embodiment of the disclosure provides a medical alarm method, which can be applied to an anesthesia device of a medical alarm system. As shown in FIG. 8, the method may include steps S121-S126 as follows.

At step S121, a preset weight setting control is displayed on a preset anesthesia interface.

At step S122, upon receiving a trigger operation for the preset weight setting control, a weight setting panel pops up on the preset anesthesia interface in response to the trigger operation.

At step S123, at least one numerical value input control is displayed on the weight setting panel, and input data is received by means of the at least one numerical value input control;

At step S124, a first preset weight threshold is determined based on the input data.

At step S125, the weight data acquired and sent by the weigher in real time is received.

At step S126, if the weight data is greater than the first preset weight threshold, it is determined to trigger an alarm and output a first alarm prompt, wherein the first alarm prompt includes at least one of sound, flashing light and interface prompt.

Accordingly, an embodiment of the disclosure provides a medical alarm method which can be applied to a weigher of a medical alarm system, the method including:
acquiring weight data of a waste gas adsorption canister by weighing in real time, and transmitting the weight data to an anesthesia device, such that the anesthesia device determines whether to trigger an alarm based on the weight data and a first preset alarm threshold, wherein the first preset alarm threshold is determined by the anesthesia device based on input data received from a preset anesthesia interface; and the alarm prompt includes at least one of sound, flashing light and an interface prompt.

It should be noted that the description of the above method embodiments is similar to the description of the above system embodiments and has similar beneficial effects as the method embodiments. For technical details which are not disclosed in the method embodiments of the disclosure, reference may be made to the description of the apparatus embodiments of the disclosure for understanding.

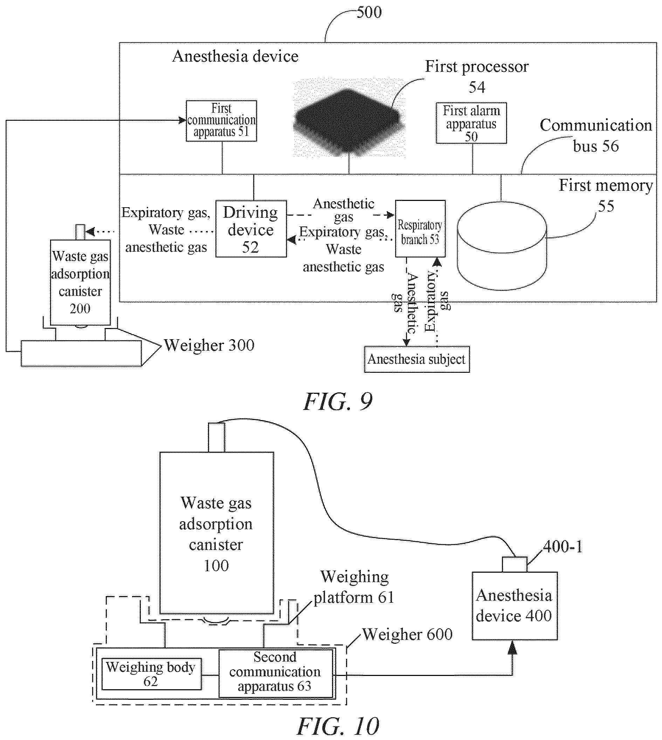

An embodiment of the disclosure provides an anesthesia device 500. As shown in FIG. 9, the anesthesia device includes a respiratory branch 53, a driving device 52, a first communication apparatus 51, a first memory 55, a first processor 54 and a first alarm apparatus 50. The driving device 52, the first communication apparatus 51, the first memory 55, the first processor 54 and the first alarm apparatus 50 are connected to one another via a communication bus 56. The respiratory branch 53 and the driving device 52 are connected to an anesthesia subject. A waste gas adsorption canister 200 is placed on a weigher 300, and an inlet of the waste gas adsorption canister 200 is connected to the driving device 52. The weigher 300 and the first communication apparatus 51 are connected to each other in a wired or wireless manner.

The driving device 52 is configured to drive an anesthetic gas to the respiratory branch 53 in an inspiratory phase.

The respiratory branch 53 is configured to deliver the anesthetic gas to the anesthesia subject in the inspiratory phase, and to receive an expiratory gas from the anesthesia subject, to obtain an unabsorbed waste anesthetic gas, and to deliver the expiratory gas and the waste anesthetic gas to the driving device 52 in an expiratory phase.

The driving device 52 is further configured to discharge the waste anesthetic gas and the expiratory gas to the waste gas adsorption canister 200 such that the waste gas adsorption canister 200 adsorbs the waste anesthetic gas and discharges the expiratory gas outside.

The first communication apparatus 51 is configured to receive weight data of the waste gas adsorption canister 200 acquired and sent by the weigher 300 in real time.

The first memory 55 is configured to store executable instructions.

The first processor 54 is configured to, when executing the executable instructions stored in the first memory 55, carry out the following steps:
determining whether to trigger an alarm based on the weight data and at least one preset alarm threshold; and upon determining to trigger an alarm, outputting an alarm prompt by means of the first alarm apparatus 50, wherein the at least one preset alarm threshold is a data criterion for an adsorption capacity abnormality of the waste gas adsorption canister or a data abnormality of the weigher, and the alarm prompt includes at least one of sound, flashing light and an interface prompt.

In some embodiments, the first communication apparatus 51 may be further configured to receive an analysis result sent by the weigher 300, wherein the analysis result is obtained by acquiring the weight data of the waste gas adsorption canister 200 in real time by the weigher 300 and performing processing and analysis based on the weight data and the at least one preset alarm threshold, wherein the at least one preset alarm threshold is a data criterion for an adsorption capacity abnormality of the waste gas adsorption canister or a data abnormality of the weigher.

The first processor 54 may further be configured to, when executing the executable instructions stored in the first memory 55, carry out the following step:
determining whether to trigger an alarm based on the analysis result, and outputting an alarm prompt upon determining to trigger an alarm, wherein the alarm prompt includes at least one of sound, flashing light and an interface prompt.

An embodiment of the disclosure provides a weigher 600. As shown in FIG. 10, the weigher includes a weighing platform 61, a weighing body 62, and a second communication apparatus 63. The weighing body 62 is connected to the second communication apparatus 63; the second communication apparatus 63 and the anesthesia device 400 are connected to each other in a wired or wireless manner; a waste anesthetic discharge port 400-1 of the anesthesia device 400 is connected to a waste gas adsorption canister 100 for discharging a waste anesthetic gas generated during anesthesia to the waste gas adsorption canister.

The weighing platform 61 is configured to place the waste gas adsorption canister 100.

The weighing body 62 is configured to acquire weight data of the waste gas adsorption canister 100 by weighing in real time.

The second communication apparatus 63 is configured to transmit the weight data to the anesthesia device 400 such that the anesthesia device 400 determines whether to trigger an alarm based on the weight data and at least one preset alarm threshold, and outputs an alarm prompt upon determining to trigger an alarm, wherein the at least one preset alarm threshold is a data criterion for an adsorption capacity abnormality of the waste gas adsorption canister or a data abnormality of the weigher.

It should be noted that in the embodiment of the disclosure, the second communication apparatus on the weigher may be an integrated circuit chip having a data transmission function, and may also be a wired or wireless network interface, and an exemplary network interface includes Bluetooth, Wireless Fidelity (WiFi), a Universal Serial Bus (USB), etc.

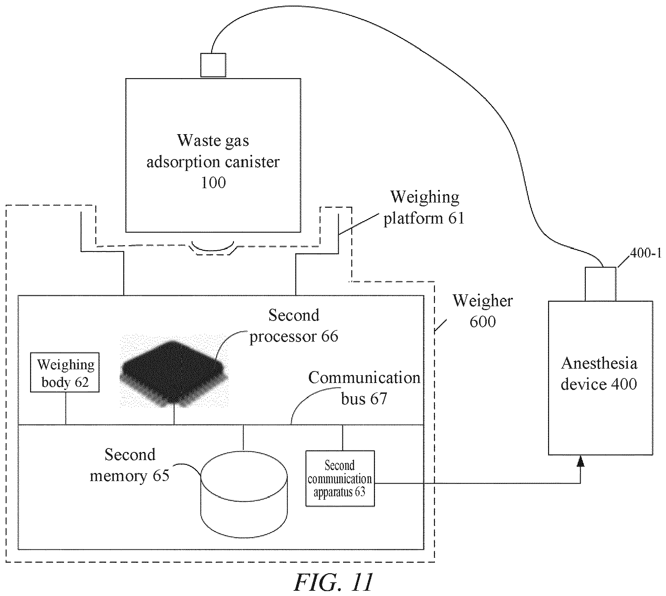

In some embodiments, as shown in FIG. 11, the weigher 600 may further include a second memory 65 and a second processor 66, wherein the second memory 65, the second processor 66, the weighing body 62 and the second communication apparatus 63 are interconnected via a communication bus 67.

The second memory 65 is configured to store executable instructions.

The second processor 66 is configured to, when executing the executable instructions stored in the second memory 65, carry out processing and analysis based on the weight data and the at least one preset alarm threshold to obtain an analysis result.

The second communication apparatus 63 is further configured to send the analysis result to the anesthesia device such that the anesthesia device determines whether to trigger an alarm based on the analysis result, wherein the at least one preset alarm threshold is a data criterion for an adsorption capacity abnormality of the waste gas adsorption canister or a data abnormality of the weigher.

An embodiment of the disclosure provides a first computer-readable storage medium that stores executable instructions, which are configured to, when executed by the first processor 54, cause the first processor to implement any medical alarm method provided in the embodiments of the disclosure.

An embodiment of the disclosure provides a second computer-readable storage medium that stores executable instructions, which are configured to, when executed by the second processor 66, cause the second processor to implement any medical alarm method provided in the embodiments of the disclosure.

Various components in the embodiments of the invention may be integrated into one processing unit, or various units may be physically present separately, or two or more units may be integrated into one unit. The integrated units described above may be implemented in the form of hardware or software functional modules.

If the integrated units are implemented in the form of the software functional modules but not sold or used as independent products, they may be stored in a computer-readable storage medium. Based on such an understanding, the technical solution of this embodiment, in essence, or its part contributing to the prior art, or all or part of the technical solution may be embodied in the form of a software product. The computer software product is stored in a storage medium and includes a number of instructions for causing a computer device (which may be a personal computer, a server, or a network device) or a processor to execute all or some of the steps of the method according to this embodiment. The foregoing storage medium includes: various media that may store program codes, such as a ferromagnetic random access memory (FRAM), a Read Only Memory (ROM), a Programmable Read-Only Memory (PROM), an EPROM (Erasable Programmable Read-Only Memory), an EEPROM (Electrically Erasable Programmable Read-Only Memory), a Flash Memory, a magnetic surface memory, a compact disc, or a Compact Disc Read-Only Memory (CD-ROM), which is not limited in the embodiments of the disclosure.

### INDUSTRIAL APPLICABILITY

The embodiments of the disclosure provide a medical alarm system, method and device, an anesthesia device, and a weigher, wherein in the medical alarm system, a weight of a waste gas adsorption canister measured on the weigher can be synchronized to the anesthesia device by means of mutual communication between the weigher and the anesthesia device, and can further be analyzed by the anesthesia device to obtain usage status of the waste gas adsorption canister in combination with at least one preset alarm threshold, to determine whether to trigger an alarm scenario corresponding to the at least one preset alarm threshold, in which a use abnormality occurs. Thus, the interconnection and intercommunication between the weigher and the anesthesia device are achieved, such that abnormal data measured by the weigher can be integrated to the anesthesia device for unified monitoring and analysis, which provides a basis for further linkage processing with the weigher based on the analysis of the abnormal data and improves the intelligence of the medical alarm system. Also, on the basis that the anesthesia device determines whether to trigger an alarm, it is further convenient to give an alarm prompt using the anesthesia device, such that alarm prompt information is more normalized and visualized, and the safety of the medical alarm system is improved.

## Claims

1. A medical alarm system, comprising an anesthesia device, a weigher and a waste gas adsorption canister; wherein the waste gas adsorption canister is connected to the anesthesia device, and the waste gas adsorption canister is placed in a preset weight measurement region of the weigher; the anesthesia device is in mutual communication with the weigher; and the anesthesia device is configured with a first alarm apparatus thereon, wherein
the anesthesia device is configured to release an anesthetic gas and to discharge a waste anesthetic gas;
the waste gas adsorption canister is configured to adsorb the waste anesthetic gas;
the weigher is configured to acquire weight data of the waste gas adsorption canister by weighing in real time and to transmit the weight data to the anesthesia device; and
the anesthesia device is configured to determine whether to trigger an alarm based on the weight data and at least one preset alarm threshold, and output an alarm prompt by means of the first alarm apparatus when it is determined to trigger an alarm; wherein the at least one preset alarm threshold is a data criterion for an adsorption capacity abnormality of the waste gas adsorption canister or a data abnormality of the weigher; and the alarm prompt comprises at least one of sound, flashing light and an interface prompt.

2. The system of claim 1, wherein the at least one preset alarm threshold comprises a first preset weight threshold; and
the anesthesia device is further configured to determine to trigger an alarm and output a first alarm prompt if the weight data is greater than the first preset weight threshold.

3. The system of claim 2, wherein the anesthesia device is configured with a human-machine interaction device thereon which comprises a display; wherein
the human-machine interaction device is configured to display a preset weight setting control on a preset anesthesia interface by means of the display, and to receive input data by means of the preset weight setting control; and
the anesthesia device is further configured to determine the first preset weight threshold based on the input data.

4. The system of claim 1 or 2, wherein the at least one preset alarm threshold comprises a second preset weight threshold; and
the anesthesia device is further configured to:
take the currently received weight data as startup weight data for each time ventilation is initiated, to determine whether the startup weight data exceeds the second preset weight threshold, wherein the second preset weight threshold is smaller than the first preset weight threshold; and
determine to trigger an alarm and output a second alarm prompt if the startup weight data is greater than the second preset weight threshold.

5. The system of any one of claims 1-4, wherein the at least one preset alarm threshold comprises a preset slope threshold; and
the anesthesia device is further configured to: record corresponding time information of acquiring the weight data; generate a slope of weight increase of the waste gas adsorption canister based on the weight data and the time information, wherein the slope of weight increase represents the trend of weight increase of the waste gas adsorption canister; acquire a current ventilation parameter in real time; and determine to trigger an alarm and output a first alarm prompt if the current ventilation parameter is not changed and the slope of weight increase is smaller than the preset slope threshold within a preset time period.

6. The system of claim 5, wherein
the anesthesia device is further configured to obtain a residual usage amount of the waste gas adsorption canister based on the first preset weight threshold and the current ventilation parameter, and to display the residual usage amount in real time by means of the display.

7. The system of claim 1 or 6, wherein the at least one preset alarm threshold comprises a preset weight range; and
the anesthesia device is further configured to determine to trigger an alarm and output a third alarm prompt if the weight data transmitted by the weigher is not received within a first preset time interval or the received weight data exceeds the preset weight range, wherein the third alarm prompt is configured to prompt that the weigher is faulty.

8. The system of any one of claims 3-7, wherein
the anesthesia device is further configured to, upon triggering an alarm, save a current alarm event in a preset alarm record list; and
the display is further configured to, upon receiving a preset view instruction, display the preset alarm record list in response to the preset view instruction.

9. The system of claim 3, wherein the preset weight setting control is a selection input control; and wherein
the human-machine interaction device is further configured to receive target specification information by means of the selection input control; and
the anesthesia device is further configured to determine a preset weight threshold corresponding to the target specification information as the first preset weight threshold according to the target specification information and based on a preset correspondence between at least one preset specification information and at least one preset weight threshold.

10. The system of any one of claims 3-9, wherein
the display is further configured to display a preset initialization control on a preset anesthesia interface;
the human-machine interaction device is further configured to, upon receiving an initialization trigger operation for the preset initialization control, send a weighing initialization instruction to the weigher in response to the initialization trigger operation; and
the weigher is further configured to, upon receiving the weighing initialization instruction, take currently acquired weight data as a reference weighing value, so as to take a weight of the waste gas adsorption canister increased based on the reference weighing value as the weight data.

11. The system of any one of claims 1-10, wherein the weigher is configured with a second alarm apparatus;
the anesthesia device is further configured to send an alarm instruction to the weigher when it is determined to trigger an alarm; and
the weigher is configured to give an alarm by means of the second alarm apparatus upon receiving the alarm instruction.

12. A medical alarm system, comprising an anesthesia device, a weigher and a waste gas adsorption canister, wherein the waste gas adsorption canister is connected to the anesthesia device, and the waste gas adsorption canister is placed in a preset weight measurement region of the weigher; the anesthesia device is in mutual communication with the weigher; and the anesthesia device is configured with a first alarm apparatus thereon, wherein
the anesthesia device is configured to release an anesthetic gas and to discharge a waste anesthetic gas;
the waste gas adsorption canister is configured to adsorb the waste anesthetic gas;
the weigher is configured to acquire weight data of the waste gas adsorption canister by weighing in real time, and to perform processing and analysis based on the weight data and at least one preset alarm threshold to obtain an analysis result, and to send the analysis result to the anesthesia device; wherein the analysis result indicates whether an adsorption capacity of the waste gas adsorption canister is abnormal or whether the data of the weigher is abnormal; and
the anesthesia device is configured to determine whether to trigger an alarm based on the analysis result, and to output an alarm prompt by means of the first alarm apparatus when it is determined to trigger an alarm, wherein the alarm prompt comprises at least one of sound, flashing light and an interface prompt.

13. The system of claim 12, wherein the at least one preset alarm threshold comprises a first preset weight threshold;
the weigher is further configured to send the analysis result indicating that the adsorption canister is overweight, to the anesthesia device, if the weight data is greater than the first preset weight threshold; and
the anesthesia device is further configured to determine to trigger an alarm and output a first alarm prompt if the analysis result is that the adsorption canister is overweight.

14. The system of claim 13, wherein the weigher is configured with a weight setting apparatus;
and the weight setting apparatus is configured to receive the first preset weight threshold.

15. The system of claim 12 or 13, wherein the at least one preset alarm threshold comprises a second preset weight threshold;
the anesthesia device is further configured to send a ventilation initiation instruction to the weigher each time ventilation is initiated;
the weigher is further configured to, upon receiving the ventilation initiation instruction, take the currently acquired weight data as startup weight data to determine whether the startup weight data exceeds the second preset weight threshold; and send the analysis result indicating that an available usage amount of the adsorption canister is insufficient, to the anesthesia device, if the startup weight data is greater than the second preset weight threshold; wherein the second preset weight threshold is smaller than the first preset weight threshold; and
the anesthesia device is further configured to determine to trigger an alarm and output a second alarm prompt if the analysis result is that an available usage amount of the adsorption canister is insufficient.

16. The system of any one of claims 12-15, wherein the at least one preset alarm threshold comprises a preset slope threshold; and
the weigher is further configured to: acquire a current ventilation parameter from the anesthesia device in real time; record corresponding time information of acquiring the weight data; generate a slope of weight increase of the waste gas adsorption canister based on the weight data and the time information, wherein the slope of weight increase represents a historical trend of weight increase of the waste gas adsorption canister; and send the analysis result indicating that the adsorption canister is overweight to the anesthesia device, if the current ventilation parameter is not changed and the slope of weight increase is smaller than the preset slope threshold within a preset time period; and
the anesthesia device is further configured to determine to trigger an alarm and output the first alarm prompt if the analysis result is that the adsorption canister is overweight.

17. The system of claim 16, wherein the anesthesia device is configured with a display;
the weigher is further configured to obtain a residual usage amount of the waste gas adsorption canister based on the first preset weight threshold and the current ventilation parameter, and to send the residual usage amount to the anesthesia device; and
the anesthesia device is further configured to display the residual usage amount in real time by means of the display.

18. The system of claim 12 or 17, wherein the at least one preset alarm threshold comprises a preset weight range;
the weigher is further configured to determine that the analysis result is that the weigher is faulty, if the acquired weight data exceeds the preset weight range; and
the anesthesia device is further configured to trigger an alarm and output a third alarm prompt, if the analysis result is not received within a second preset time interval or the analysis result is that the weigher is faulty.

19. The system of any one of claims 14-18, wherein
the anesthesia device is further configured to, upon triggering an alarm, save a current alarm event in a preset alarm record list; and
the display is further configured to, upon receiving a preset view instruction, display the preset alarm record list in response to the preset view instruction.

20. The system of claim 14, wherein an information input control is preset on the weight setting apparatus;
the information input control is configured to receive target specification information; and
the weigher is further configured to determine a preset weight threshold corresponding to the target specification information as the first preset weight threshold according to the target specification information and based on a preset correspondence between at least one term of preset specification information and at least one preset weight threshold.

21. The system of any one of claims 12-20, wherein the weigher is configured with a preset initialization button, and
the weigher is further configured to, upon receiving a trigger operation for the preset initialization button, take currently acquired weight data as a reference weighing value in response to the trigger operation, so as to take a weight of the waste gas adsorption canister increased based on the reference weighing value as the weight data.

22. The system of any one of claims 12-21, wherein the weigher is configured with a second alarm apparatus; and
the second alarm apparatus is configured to output an alarm prompt if the analysis result is any one of the adsorption canister being overweight, the available usage amount of the adsorption canister being insufficient and the weigher being faulty.

23. A medical alarm method, comprising:
receiving weight data acquired and sent by a weigher in real time; and
determining whether to trigger an alarm based on the weight data and at least one preset alarm threshold, and outputting an alarm prompt when it is determined to trigger an alarm, wherein the alarm prompt comprises at least one of sound, flashing light and an interface prompt, and the at least one preset alarm threshold is a data criterion for an adsorption capacity abnormality of a waste gas adsorption canister or a data abnormality of the weigher.

24. A medical alarm method, comprising:
displaying a preset weight setting control on a preset anesthesia interface;
upon receiving a trigger operation for the preset weight setting control, a weight setting panel popping up on the preset anesthesia interface in response to the trigger operation;
displaying at least one numerical value input control on the weight setting panel, and receiving input data by means of the at least one numerical value input control;
determining a first preset weight threshold based on the input data;
receiving weight data acquired and sent by a weigher in real time; and
determining to trigger an alarm and outputting a first alarm prompt if the weight data is greater than the first preset weight threshold, wherein the first alarm prompt comprises at least one of sound, flashing light and an interface prompt.

25. A medical alarm method, comprising:
acquiring weight data of a waste gas adsorption canister by weighing in real time, and transmitting the weight data to an anesthesia device, such that the anesthesia device determines whether to trigger an alarm based on the weight data and at least one preset alarm threshold, and outputs an alarm prompt when it is determined to trigger an alarm, wherein the at least one preset alarm threshold is a data criterion for an adsorption capacity abnormality of the waste gas adsorption canister or a data abnormality of a weigher; and the alarm prompt comprises at least one of sound, flashing light and an interface prompt.

26. An anesthesia device, comprising a respiratory branch, a driving device, a first communication apparatus, a first memory, a first processor and a first alarm apparatus, wherein
the driving device is configured to drive an anesthetic gas to the respiratory branch in an inspiratory phase;
the respiratory branch is configured to deliver the anesthetic gas to an anesthesia subject in the inspiratory phase; to receive an expiratory gas from the anesthesia subject, to obtain an unabsorbed waste anesthetic gas, and to deliver the expiratory gas and the waste anesthetic gas to the driving device in an expiratory phase;
the driving device is further configured to discharge the waste anesthetic gas and the expiratory gas to a waste gas adsorption canister such that the waste gas adsorption canister adsorbs the waste anesthetic gas and discharges the expiratory gas outside;
the first communication apparatus is configured to receive weight data of the waste gas adsorption canister acquired and sent by a weigher in real time;
the first memory is configured to store executable instructions; and
the first processor is configured to, when executing the executable instructions stored in the first memory, carry out the following steps:
determining whether to trigger an alarm based on the weight data and at least one preset alarm threshold; and outputting an alarm prompt by means of the first alarm apparatus when it is determined to trigger an alarm, wherein the at least one preset alarm threshold is a data criterion for an adsorption capacity abnormality of the waste gas adsorption canister or a data abnormality of the weigher, and the alarm prompt comprises at least one of sound, flashing light and an interface prompt.

27. A weigher, comprising a weighing platform, a weighing body and a second communication apparatus, wherein
the weighing platform is configured to place a waste gas adsorption canister;
the weighing body is configured to acquire weight data of the waste gas adsorption canister in real time; and
the second communication apparatus is configured to transmit the weight data to an anesthesia device such that the anesthesia device determines whether to trigger an alarm based on the weight data and at least one preset alarm threshold, and outputs an alarm prompt when it is determined to trigger an alarm, wherein the at least one preset alarm threshold is a data criterion for an adsorption capacity abnormality of the waste gas adsorption canister or a data abnormality of the weigher.
